# EUROPÄISCHE PATENTSCHRIFT

(11) **EP 2 288 400 B1**
(45) Veröffentlichungstag und Bekanntmachung des Hinweises auf die Patenterteilung: **19.09.2012**
(21) Anmeldenummer: 08759813.2
(22) Anmeldetag: 20.05.2008
(51) Int. Cl.: A61M 5/315

(54) **VORRICHTUNG ZUR VERABREICHUNG EINES INJIZIERBAREN PRODUKTS MIT RESTMENGENANZEIGE**
DEVICE FOR ADMINISTERING AN INJECTABLE PRODUCT COMPRISING A RESIDUAL AMOUNT DISPLAY
DISPOSITIF D'ADMINISTRATION D'UN PRODUIT INJECTABLE COMPORTANT UNE INDICATION DE QUANTITÉ RÉSIDUELLE

(43) Veröffentlichungstag der Anmeldung: 02.03.2011
(73) Patentinhaber: Tecpharma Licensing AG, 3401 Burgdorf (CH)
(72) Erfinder: KIRCHHOFER, Fritz, CH-3454 Sumiswald (CH); SELZ, Anjan, CH-3006 Bern (CH)
(86) Internationale Anmeldenummer: PCT/EP2008/056205
(87) Internationale Veröffentlichungsnummer: WO 2009/141005

(56) Entgegenhaltungen:
- EP-A- 0 554 996
- WO-A-01/72361
- WO-A-97/36626
- WO-A-2006/130098
- DE-A1- 4 208 677
- DE-A1-102006 038 123

## Beschreibung

Die Erfindung betrifft eine Vorrichtung zur Verabreichung eines injizierbaren Produkts mit Restmengenanzeige. Die Vorrichtung ist vorzugsweise ein Injektionsgerät, kann grundsätzlich aber auch ein Infusionsgerät sein. Die Erfindung zielt auf eine Verwendung im Bereich der Selbstverabreichung ab, also auf Anwendungen, in denen sich der jeweilige Benutzer das Produkt selbst verabreicht. Die Erfindung ist jedoch auch für die Verabreichung durch medizinisch geschultes Personal von Vorteil und nicht auf die Selbstverabreichung beschränkt.

In der Selbstverabreichung muss mehr noch als bei Verabreichung durch medizinisch geschultes Personal gewährleistet sein, dass das Produkt auf einfache Weise und in korrekter Dosierung verabreicht wird. In der Diabetestherapie beispielsweise ist die Selbstverabreichung von Insulin üblich. Zu bedenken ist, dass die Geschicklichkeit der Selbstverabreicher in weiten Grenzen schwankt. Wird das Produkt, wie beispielsweise in der Diabetestherapie über längere Zeiträume immer wieder verabreicht, stellt sich ein Übungseffekt ein. In anderen Therapien mit zum Teil nur wenigen Verabreichungen kann sich ein solcher Übungseffekt nicht einstellen. Umso mehr kommt es auf eine einfache und sichere Bedienbarkeit des jeweiligen Geräts an. Die Problematik wird noch verschärft, wenn die zu verabreichende Produktdosis veränderbar sein soll, sei es dass ein Arzt die Produktdosis jeweils für einen bestimmten Selbstverabreicher voreinstellt oder der Selbstverabreicher selbst die Produktdosis auswählt.

Aus der DE 102 32 410 B4 ist eine Vorrichtung bekannt, die auf einfache Weise die Einstellung der zu verabreichenden Produktdosis individuell ermöglicht. Die Vorrichtung weist ein Dosierglied auf, das zur Einstellung unterschiedlicher Produktdosen in unterschiedliche Auswahlpositionen verstellbar ist. Das Dosierglied ist in einem Gewindeeingriff mit einem Gehäuse der Vorrichtung um eine Drehachse drehbar und axial bewegbar. Das Gehäuse ist in einem durchsichtigen Gehäuseabschnitt um die Drehachse spiralig umlaufend mit einer Dosisskala versehen. Das Dosierglied bildet einen Marker, an dessen Position relativ zu der Dosisskala des Gehäuses die eingestellte Dosis ablesbar ist. Für die Förderung des Produkts umfasst die Vorrichtung eine Kolbenstange, die in eine Vortriebsrichtung bewegbar ist, und ein Abtriebsglied, das relativ zu dem Dosierglied gegen die Vortriebsrichtung einen Dosierhub bis in eine von dem Dosierglied vorgegebene Dosisposition und aus der Dosisposition einen Ausschütthub in die Vortriebsrichtung ausführen kann, wobei das Abtriebsglied mit der Kolbenstange so gekoppelt ist, dass es den Dosierhub relativ zu der Kolbenstange ausführt und der Ausschütthub die Bewegung der Kolbenstange in die Vortriebsrichtung bewirkt. Das Abtriebsglied bildet einen zweiten Marker, dessen axiale Position relativ zu dem Gehäuse durch den durchsichtigen Gehäuseabschnitt hindurch an der Dosisskala ablesbar ist und die aus einem Reservoir der Vorrichtung noch ausschüttbare Produktmenge anzeigt.

Aus der WO 97/36626 A ist ein Injektionsgerät bekannt, mit einer in einem Ampullenhalter gehaltenen Ampulle, einer Dosisvorwähleinrichtung und einer Abgabeeinrichtung für die eingestellte Dosis. Die Abgabeeinrichtung weist eine Schubstange auf, die mit einer Dosierhülse gekoppelt ist, wobei die Dosierhülse beim Einstellen der Dosis relativ zu der Schubstange verstellt wird und zum Ausschütten die Dosierhülse die Schubstange mit in die Ausschüttrichtung nimmt. Das Injektionsgerät weist zwei Skalen auf, eine erste, an der die eingestellte Dosis abgelesen werden kann und eine zweite, die die noch abgebbare Restmenge nach der Injektion anzeigt. Die EP 0 554 996 A1 beschreibt einen Injektionspen mit einer Dosiervorrichtung, mit der eine Dosis eingestellt und die eingestellte Dosis angezeigt werden kann. Das Medikament zur Injektion wird in einer Ampulle in den Injektionspen eingegeben. Ein Ampullenwechsel kann schnell und einfach durchgeführt werden. Die DE 10 2006 038 123 A1 beschreibt eine Injektionsvorrichtung, die eine robuste und einfache Einstellung einer fixed-dose erlaubt. Dazu weist die Injektionseinrichtung ein Bedienelement oder Koppelelement auf, das vor dem Einbringen einer Ampulle in die Injektionseinrichtung in einer ersten Halteverbindung in einer ersten Position gehalten werden kann und nach dem Einbringen der Ampulle in eine zweite axial zur ersten Position versetzten zweiten Position verschoben werden kann, wo das Bedienelement oder Koppelelement von einer zweiten Halteverbindung gehalten werden kann. Aus der WO 01/72361 A1 ist eine Injektionsvorrichtung mit einer Anzeige für eine eingestellte Abgabedosis bekannt. Die Mechanik zum Einstellen und Anzeigen der eingestellten Dosis ist so ausgebildet, dass keine Dosis eingestellt werden kann, die größer ist, als die Menge des abzugebenden Medikaments, die beim Einstellen der Dosis noch zur Verfügung steht. Die DE 42 08 677 A1 beschreibt eine Injektionsvorrichtung, insbesondere für sehgeschädigte Nutzer, mit großflächigen Bedienelementen zur Dosierungsvorwahl, um eine verbesserte visuelle und taktil erkennbare Skalenbeschriftung zu ermöglichen. Aus der WO 2006/130098 A1 ist eine Vorrichtung zur Abgabe eines Medikaments bekannt, mit der mit hoher Genauigkeit eine voreinstellbare Menge eines Medikaments abgegeben werden kann und die dem Nutzer anzeigt, wie viel Medikament am Ende der Injektion noch zur Verfügung steht.

Es ist eine Aufgabe der Erfindung, bei einer Vorrichtung der genannten Art eine Restmenge des Produkts, die aus einem Produktreservoir noch ausschüttbar ist, einfach und eindeutig anzuzeigen.

Die Erfindung hat eine Vorrichtung zur Verabreichung eines injizierbaren Produkts zum Gegenstand, die ein Gehäuse mit einer Aufnahme für das Produkt, ein Dosierglied zur Einstellung einer zu verabreichenden Produktdosis und eine Fördereinrichtung für die Förderung der eingestellten Produktdosis umfasst. Das Dosierglied ist zur Einstellung unterschiedlicher Produktdosen relativ zu dem Gehäuse in unterschiedliche Auswahlpositionen verstellbar. Das Gehäuse kann unmittelbar ein Reservoir für das Produkt bilden, bevorzugter bildet jedoch ein in der Aufnahme des Gehäuses aufgenommenes oder aufnehmbares Behältnis das Produktreservoir. Solch ein Behältnis kann insbesondere als Ampulle gebildet sein. Derartige Behältnisse sind aus unterschiedlichen Therapien, beispielsweise der Diabetestherapie, bekannt. Das Produkt wird mittels eines Kolbens gefördert, der in dem Reservoir in eine Vortriebsrichtung bewegbar aufgenommen ist. Falls ein Produktbehältnis in der Aufnahme des Gehäuses eingesetzt ist oder die Aufnahme selbst das Reservoir bildet, ist der Kolben Bestandteil der Fördereinrichtung. Die Fördereinrichtung umfasst eine Kolbenstange für solch einen Kolben, die zur Ausschüttung der Produktdosis in die Vortriebsrichtung bewegbar ist. Die Kolbenstange ist vorzugsweise eine stabförmige, in sich steife Struktur. Sie ist vorzugsweise in einem Stück gefertigt, kann aber grundsätzlich auch aus mehreren Teilen zusammengesetzt sein, beispielsweise auch aus kettenartig miteinander beweglich verbundenen Schubgliedern. Sie muss eine in die Vortriebsrichtung wirkende Kraft auf den Kolben übertragen können.

Die Fördereinrichtung umfasst ferner ein Abtriebsglied, dass gegen die Vortriebsrichtung relativ zu dem Gehäuse, vorzugsweise auch relativ zu dem Dosierglied, einen Dosierhub bis in eine mittels des Dosierglieds vorgegebene Dosisposition und aus dieser Dosisposition einen Ausschütthub in die Vortriebsrichtung ausführen kann. Das Abtriebsglied ist mit der Kolbenstange so gekoppelt, dass es den Dosierhub relativ zu der Kolbenstange ausführt und bei seinem Ausschütthub die Bewegung der Kolbenstange in die Vortriebsrichtung bewirkt. Vorzugsweise sind die Kolbenstange und das Abtriebsglied so miteinander gekoppelt, das Abtriebsglied relativ zu der Kolbenstange bei jedem Dosierhub ein Stück weiter gegen die Vortriebsrichtung bewegt wird und dadurch die Länge der Kolbenstange, gemessen zwischen einem in Vortriebsrichtung vorderen Ende der Kolbenstange und einem Koppeleingriff der Kolbenstange, vorzugsweise einem Koppeleingriff mit unmittelbar dem Abtriebsglied, vergrößert wird.

Die Verabreichungsvorrichtung umfasst eine Restmengenanzeige zum Anzeigen der durch den nächsten Ausschütthub noch ausschüttbaren Menge des Produkts. Ist die nach Ausführung eines oder mehrerer Ausschütthübe im Reservoir verbliebene Produktmenge größer oder wenigstens so groß wie die mit dem aktuellen Dosierhub eingestellte Produktdosis, zeigt die Restmengenanzeige vorzugsweise die eingestellte Produktdosis an, alternativ kann sie aber auch so ausgebildet sein, dass sie dem Benutzer nur signalisiert, vorzugsweise optisch, dass noch eine für die Verabreichung der eingestellten Produktdosis ausreichende Produktmenge im Reservoir zur Verfügung steht. Die Restmengenanzeige weist eine Dosisskala auf, die mit einer einzigen oder mehreren symbolhaften Dosismarke(n) gebildet sein kann, beispielsweise eine schlichte Farbanzeige mit einer beispielsweise grünen Farbmarke. Bei Verwendung nur von Farbmarken ist allerdings eine Anzeige der Restmenge nur sinnvoll, wenn nur wenige Auswahlmöglichkeiten hinsichtlich der Dosis gegeben sind oder nur eine einzige Dosis von einem Arzt voreingestellt wird. Bevorzugt wird die noch verfügbare Produktmenge jedoch als Zahlenwert, besonders bevorzugt als Dosiswert in Dosiseinheiten angezeigt. Unterschreitet die noch verfügbare Produktmenge die eingestellte Produktdosis, zeigt die Restmenganzeige dies an, beispielsweise in Form eines Warnsignals. Das Warnsignal kann akustisch oder vibratorisch ausgegeben werden, bevorzugt wird es optisch ausgegeben oder umfasst zumindest eine optische Ausgabe. Ein optisches Warnsignal kann in dem Sichtbarwerden einer schlichten Farbmarkierung, beispielsweise einer roten Marke, in einem Sichtfenster ausgegeben werden. Bevorzugt wird jedoch die noch verfügbare Restmenge als Zahlenwert, besonders bevorzugt als Dosiswert in Dosiseinheiten, angezeigt.

Die Restmengenanzeige umfasst wenigstens zwei Restmengenglieder, die relativ zueinander beweglich sind und durch die Position, die sie relativ zueinander einnehmen, die noch verfügbare Restmenge anzeigen. Die wenigstens zwei Restmengenglieder werden im folgenden als erstes Restmengenglied und zweites Restmengenglied bezeichnet. Die Relativbeweglichkeit kann so gestaltet sein, dass jedes der Restmengenglieder relativ zu dem Gehäuse beweglich ist. In bevorzugten Ausführungen ist nur das erste Restmengenglied relativ zu dem Gehäuse beweglich, das zweite Restmengenglied kann vorteilhafterweise von dem Gehäuse gebildet werden, beispielsweise von einer äußeren Gehäuseschale, die Komponenten der Vorrichtung umgibt. Als Gehäuse werden auch Einbauteile oder Anbauteile solch einer Gehäuseschale verstanden. Das erste Restmengenglied ist mit dem Abtriebsglied so gekoppelt, dass der Dosierhub eine Bewegung des ersten Restmengenglieds in eine erste Richtung und der Ausschütthub des Abtriebsglieds eine Bewegung in die Gegenrichtung bewirkt. Eines der Restmengenglieder weist die Dosisskala mit einer oder vorzugsweise mehreren Dosismarken auf, vorzugsweise das erste Restmengenglied. Die Restmengenglieder wirken so zusammen, dass aus der Position, die das andere der Restmengenglieder relativ zu der Dosisskala einnimmt, die mit dem nächsten Ausschütthub noch ausschüttbare Restmenge des Produkts ablesbar ist. Das andere Restmengenglied bildet einen Marker für die Dosisskala. Der Marker kann insbesondere als Sichtfenster gebildet sein, durch das die Dosismarke der Skala, die der noch verfügbaren Restmenge zugeordnet ist, vorzugsweise als Zahlenwert entspricht, ablesbar ist. Das Sichtfenster ist vorzugsweise im Gehäuse und dort vorzugsweise als Durchbruch gebildet. Der Durchbruch kann offen sein oder von einer durchsichtigen Scheibe abgedeckt werden, wobei die Scheibe zu einer Lupe weitergebildet sein kann. Die Dosisskala kann wie gesagt eine Farbskala mit wenigen, gegebenenfalls nur zwei oder drei unterschiedlichen Farben, beispielsweise grün und rot oder grün, gelb und rot, sein. Bevorzugt handelt es sich jedoch um eine Dosisskala mit zahlenmäßig in Dosiseinheiten angegebenen Dosiswerten, die in die erste Richtung der Relativbeweglichkeit des ersten Restmengenglieds dem Wert nach zunehmen.

Der Ort, an dem die Restmenge angezeigt wird, ist relativ zu dem Gehäuse vorzugsweise unveränderlich, d.h. die Restmengenanzeige ist in derartigen Ausführungen relativ zum Gehäuse ortsfest. In bevorzugten Ausführungen weist daher das durch den Dosierhub und den Ausschütthub bewegte erste Restmengenglied die Dosisskala auf, und das zweite Restmengenglied bildet einen ortsfesten Marker für die Dosisskala.

Die Verabreichungsvorrichtung kann mit Vorteil zusätzlich zur Restmengenanzeige eine Dosisanzeige aufweisen, die stets die eingestellte Produktdosis anzeigt. Die Dosisskala der Restmengenanzeige kann gleichzeitig auch eine Dosisskala für die weitere Dosisanzeige bilden. Eine für die Restmengenanzeige und die weitere Dosisanzeige gemeinsame Dosisskala kann am einfachsten noch in solchen Ausführungen verwirklicht werden, in denen nicht das bewegliche erste Restmengenglied, sondern das zweite Restmengenglied mit der Dosisskala versehen ist und von dem Gehäuse gebildet wird. In bevorzugten Ausführungen ist für die weitere Dosisanzeige jedoch eine eigene, weitere Dosisskala vorgesehen. Ein besonderer Vorteil der Anzeige der Restmenge und der eingestellten Produktdosis mittels jeweils einer eigenen Dosisskala ist, das der Ort, an dem die Restmenge angezeigt wird und der Ort, an dem die eingestellte Produktdosis angezeigt wird, frei gewählt werden können. Sind ferner die beiden Dosisskalen relativ zum Gehäuse beweglich, ist dies für die Ablesbarkeit und somit die einfache und sichere Handhabung der Verabreichungsvorrichtung besonders vorteilhaft. So können die beiden Anzeigen insbesondere so angeordnet sein, dass sie stets gleichzeitig und am gleichen Ort, bezogen auf das Gehäuse, abgelesen und somit vom Benutzer gleichzeitig erfasst werden können.

Bevorzugt sind die beiden Anzeigen längs einer zu der Vortriebsrichtung parallelen Achse voneinander beabstandet an der gleichen Längsseite der Vorrichtung angeordnet, so dass die beiden Anzeigen sicher und rasch mit einem Blick erfasst werden können. Bei bezüglich des Gehäuses ortsfester Anordnung muss der Benutzer vorteilhafterweise auch nicht das Gehäuse bewegen, sondern kann dieses bei der Auswahl der Dosis und entsprechend bei dem Ablesen der Anzeigen mit nur einer Hand halten. Die Bedienung bei der Dosisauswahl ist daher einfach und nicht zuletzt deshalb auch sicher. Die Dosisanzeige für die eingestellte Produktdosis weist ebenfalls einen Marker auf, der vorzugsweise von einem Sichtfenster und besonders bevorzugt von einem Sichtfenster des Gehäuses gebildet wird. Für dieses Sichtfenster gelten sinngemäß die Ausführungen zum Sichtfenster der Restmengenanzeige.

In einer Weiterentwicklung weist die Verabreichungsvorrichtung im Bereich der Produktaufnahme eine weitere Restmengenanzeige auf, indem das Gehäuse im Bereich der Aufnahme durchsichtig ist, wobei die Durchsichtigkeit vorzugsweise mittels eines Sichtfensters gebildet ist, so dass der Blick auf den das Produkt fördernden Kolben frei ist. Falls die Aufnahme des Gehäuses dafür eingerichtet ist, ein mit dem Produkt gefülltes Behältnis, vorzugsweise eine vorabgefüllte Ampulle mit dem Kolben, aufzunehmen, ist das Behältnis in derartigen Ausführungen ebenfalls durchsichtig oder zumindest bereichsweise durchsichtig, um den Kolben erkennen zu können. Im Bereich der Aufnahme erstreckt sich in Vortriebsrichtung eine Dosisskala, so dass im durchsichtigen Bereich die axiale Position des Kolbens relativ zu dieser Dosisskala erkennbar ist und der Kolben daher für die Dosisskala einen Marker bildet, dessen Position an der weiteren Dosisskala die noch aus dem Reservoir ausschüttbare Restmenge des Produkts anzeigt. Der Kolben kann für die Zwecke des Anzeigens mit einer Markierung versehen sein. In bevorzugten einfachen Ausführungen dient jedoch eine Kante des Kolbens, vorzugsweise eine in Vortriebsrichtung vordere Kante, als Markierung für die Dosisskala. Die Dosisskala wird vorzugsweise von zahlenmäßigen Dosiswerten in Dosiseinheiten gebildet, die axial hintereinander und dem Wert nach in Vortriebsrichtung fallend längs des Weges des Kolbens angeordnet sind. Eine derart weiterentwickelte Verabreichungsvorrichtung umfasst somit eine Restmengenanzeige, die mit jeder Dosisauswahl eingestellt und mit der Ausschüttung der eingestellten Dosis zurückgesetzt wird, somit die mit jeder einzelnen Ausschüttung ausschüttbare Restmenge anzeigt, und die weitere Restmengenanzeige, die stets die insgesamt im Reservoir noch verbliebene Restmenge anzeigt. Die im Bereich der Aufnahme erstreckte Dosisskala deckt vorzugsweise zwar die insgesamt im vollen Reservoir befindlichen Produktmenge ab und erstreckt sich in solchen Ausführungen über den vom Kolben insgesamt maximal zurücklegbaren Weg. Grundsätzlich ist es jedoch auch möglich, dass diese Dosisskala nur mit der Produktmenge korrespondiert, die mit einem längsten Ausschütthub des Abtriebsglieds ausschüttbar ist. In derartigen Ausführungen, erstreckt sich die Dosisskala nur über das in Vortriebsrichtung letzte Wegstück, dass der Kolben im Reservoir im Zuge eines für das Abtriebsglied maximalen Ausschütthubs zurücklegt. In derartigen Ausführungen kann das Gehäuse im Bereich der Aufnahme in einem der Länge des maximalen Ausschütthubs entsprechend axial kurzen Abschnitt durchsichtig sein, beispielsweise nur ein im Vergleich zu den bevorzugten Ausführungen entsprechend kurzes Sichtfenster aufweisen.

In einer nicht beanspruchten Variante ist das erste Restmengenglied mit dem Abtriebsglied axial nicht beweglich gekoppelt, so dass es dessen Dosierhub mitmacht. Es kann mit dem Abtriebsglied in einem Stück geformt oder insbesondere an dem Abtriebsglied axial nicht beweglich befestigt sein. Solch ein erstes Restmengenglied kann vorteilhafterweise in die vom Gehäuse für das Produkt gebildete Aufnahme ragen.

Das erste Restmengenglied und das Abtriebsglied werden mittels eines Getriebes miteinander gekoppelt, das bevorzugt den Dosierhub und den Ausschütthub des Abtriebsglieds in Bezug auf die Länge übersetzt. Der Dosierhub und der Ausschütthub des Abtriebsglieds erzeugen daher eine entsprechend der Getriebeübersetzung längere Hubbewegung des ersten Restmengenglieds in die erste Richtung und die zweite Richtung. Ein Vorteil solch einer Übersetzung ist, dass die Dosisskala der Restmengenanzeige, mit der vorzugsweise das erste Restmengenglied versehen ist, die stattdessen aber auch beim zweiten Restmengenglied vorgesehen sein kann, über eine größere Länge als der Dosier- und der Ausschütthub erstreckt ist. Dosismarken der Dosisskala können dementsprechend größer oder in größeren Abständen voneinander angeordnet sein als bei einer Dosisskala mit der Länge des Ausschütthubs. Das erste Restmengenglied und das Abtriebsglied können die einzigen Getriebeglieder des Getriebes bilden, wie dies bevorzugt wird. Das Getriebe kann aber auch ein oder mehrere Getriebezwischenglieder aufweisen, um die Hubbewegungen des Abtriebsglieds in die Hubbewegungen des ersten Restmengenglieds umzuwandeln.

Obgleich einem Übersetzungsgetriebe der Vorzug gegeben wird, soll jedoch nicht ausgeschlossen sein, dass das Abtriebsglied mittels eines Untersetzungsgetriebes mit dem ersten Restmengenglied gekoppelt ist. Ein Untersetzungsgetriebe kommt am ehesten dann in Frage, wenn die Dosisskala der Restmengenanzeige nur wenige Dosismarken, beispielsweise Farbmarken, aufweist oder aufgrund beengter Platzverhältnisse nur wenig Spielraum für die Bewegungen des ersten Rastmengenglieds vorhanden ist.

Das Getriebe kann so ausgebildet sein, dass die Richtung der Hubbewegungen des Abtriebsglieds und die Hubbewegungen des ersten Restmengenglieds zueinander parallel sind. Die Hubbewegungen können insbesondere gleich gerichtet sein. Allerdings werden die Hübe des Abtriebsglieds untersetzt oder vorzugsweise der Länge nach übersetzt.

Das Getriebe kann besonders vorteilhaft jedoch so gebildet sein, dass die Hubbewegungen des ersten Restmengenglieds eine Querrichtungskomponente zu den Hubbewegungen des Abtriebsglieds aufweisen. Die Hubbewegungen des ersten Restmengenglieds können insbesondere einfach nur quer zu den Hubbewegungen des Abtriebsglieds gerichtet sein. Besonders bevorzugt werden die Hubbewegungen des Abtriebsglieds mittels des Getriebes in Drehbewegungen des ersten Restmengenglieds umgewandelt. Der Drehbewegung des ersten Restmengenglieds kann eine Translationsbewegung längs der Drehachse überlagert sein, vorzugsweise führt das erste Restmengenglied jedoch nur Drehbewegungen um die Drehachse aus. Die Drehachse kann in beiden Ausführungen parallel zu einer Translationsachse des Abtriebsglieds weisen, insbesondere können die Translationsachse des Abtriebsglieds und die Drehachse des ersten Restmengenglieds identisch sein.

Das Getriebe ist als Kurvengetriebe ausgeführt. Das Kurvengetriebe umfasst ein Führungsglied mit einer Führungskurve und ein Eingriffsglied mit einem mit der Führungskurve in einem Führungseingriff befindlichen Eingriffselement. Das Kurvengetriebe kann mehrere Führungsglieder oder mehrere Eingriffsglieder, also auch mehrere Führungsglieder und mehrere Eingriffsglieder aufweisen, die jeweils paarweise in einem Führungseingriff sind. Bevorzugter wandelt das Kurvengetriebe die Bewegungen des Abtriebsglieds nur in einem einzigen Führungseingriff in die Bewegungen des ersten Restmengenglieds um. In derartigen Ausführungen bildet eines aus Abtriebsglied und erstem Restmengenglied das Führungsglied und das andere das Eingriffsglied des Kurvengetriebes. Vorzugsweise bildet das erste Restmengenglied das Führungsglied mit der Führungskurve. Das Führungsglied und das Eingriffsglied können insbesondere ein Schraubgelenk miteinander bilden. Die Führungskurve erstreckt sich dementsprechend längs einer Schraubenlinie um eine Drehachse des Schraubgelenks. Das Eingriffsglied kann ein einfacher Eingriffsnocken oder eine zweite Führungskurve sein, die über einen entsprechend längeren Führungsabschnitt mit der anderen Führungskurve passend geformt ist. Vorteilhafterweise ist die Schraubensteigung des Schraubgelenks so groß, dass im Führungseingriff keine Selbsthemmung auftritt, wenn eines aus Führungsglied und Eingriffsglied relativ zu dem anderen axial oder in Umfangsrichtung bewegt wird. Eine Relativbewegung in Umfangsrichtung erzeugt eine Relativbewegung in Achsrichtung, ebenso erzeugt eine Relativbewegung in Achsrichtung eine Relativbewegung in Umfangsrichtung. Sind die Hubbewegungen des Abtriebsglieds axiale Translationsbewegungen und das Abtriebsglied und das erste Restmengenglied miteinander in dem Führungseingriff, wie dies bevorzugt wird, erzeugen die Hubbewegungen des Abtriebsglieds daher Hubdrehbewegungen des ersten Restmengenglieds, das hierfür vorteilhafterweise frei hin und her drehbar gelagert ist.

Die Vorrichtung weist in einer Weiterentwicklung einen Ausschüttungszähler auf, der die Anzahl der Produktausschüttungen zählt und zur Anzeige bringt. Für vorteilhafte Ausgestaltungen des Ausschüttungszählers als solchen und insbesondere in Kombination mit der Restmengenanzeige wird die internationale Anmeldung der Anmelderin vom heutigen Tage mit dem Titel "Vorrichtung zur Verabreichung eines injizierbaren Produkts mit Ausschüttungszähler" in Bezug genommen.

In bevorzugten Ausführungen umfasst die Fördereinrichtung ein Antriebsglied, das gemeinsam mit oder vorzugsweise relativ zu dem Dosierglied axiale Hubbewegungen zwischen einer Ausschüttposition und einer Auslöseposition ausführen kann. Der Hub des Antriebsglieds weist für wenigstens eine der Auswahlpositionen des Dosierglieds eine andere axiale Länge auf als der Dosier- oder Ausschütthub des Abtriebsglieds. Eine Kopplung, die das Antriebsglied mit dem Abtriebsglied koppelt, ist so eingerichtet, dass auch für die wenigstens eine der Auswahlpositionen, in der die Hübe des Antriebsglieds und des Abtriebsglieds unterschiedlich lang sind, der Hub des Antriebsglieds in die Auslöseposition den Dosierhub des Abtriebsglieds und der Hub des Antriebsglieds in die Ausschüttposition den Ausschütthub des Abtriebsglieds bewirkt. Die Kopplung gleicht somit die Längenunterschiede der Hübe des Antriebsglieds und des Abtriebsglieds aus.

Indem das Antriebsglied und das Abtriebsglied relativ zu dem Dosierglied bewegbar sind und ferner das Antriebsglied für wenigstens eine der Auswahlpositionen des Dosierglieds relativ zu dem Abtriebsglied auf solch eine Weise bewegbar ist, dass es wenigstens einen Teil seines Hubs bei axial ruhendem, d. h. nicht bewegtem Abtriebsglied ausführen kann und in diesem Sinne unabhängig von dem Abtriebsglied bewegbar ist, werden die axialen Längen der Hübe des Abtriebsglieds und des Antriebsglieds voneinander entkoppelt, so dass der Hub des Antriebsglieds seiner axialen Länge nach optimiert werden kann. Die Kopplung von Abtriebsglied und Antriebsglied ist entsprechend dafür eingerichtet, die genannten Relativbeweglichkeiten zu ermöglichen, andererseits aber sicherzustellen, dass der jeweilige Hub des Antriebsglieds den jeweils zugeordneten Hub des Abtriebsglieds bewirkt, der Hub in die Auslöseposition dementsprechend den Dosierhub und der Hub in die Ausschüttposition den Ausschütthub. Insbesondere kann die axiale Länge des Hubs des Antriebsglieds für unterschiedliche Auswahlpositionen des Dosierglieds, vorzugsweise für sämtliche Auswahlpositionen des Dosierglieds, jeweils die gleiche sein. Dies ist für die Handhabung von Vorteil, da beim Benutzer Unsicherheit bezüglich der Korrektheit der Dosiseinstellung und -verabreichung vermieden wird. Vortelhafterweise ist der Hub des Antriebsglieds für jede Auswahlposition des Dosierglieds wenigstens so lang wie der längste Hub des Abtriebsglieds. Dies gilt sowohl für Ausführungen, in denen die Länge des Hubs des Antriebsglieds variiert, als auch für die bevorzugt gleichlangen Hübe des Antriebsglieds. Vorzugsweise sind die variablen Hübe oder ist der bevorzugt konstant lange Hub des Antriebsglieds länger als der längste Hub des Abtriebsglieds.

Das Antriebsglied bildet in bevorzugten Ausführungen gleichzeitig auch ein Betätigungsglied der Vorrichtung. In derartigen Ausführungen ragt es aus dem Gehäuse, vorzugsweise entgegen der Vortriebsrichtung, so dass der Benutzer es mit der Hand greifen und bevorzugt mittels Zugbewegung in die Auslöseposition überführen kann. Die Hubbewegung in die Auslöseposition ist vorteilhafterweise eine reine Translationsbewegung, grundsätzlich könnte der Translationsbewegung aber auch eine Rotationsbewegung um die Translationsachse überlagert sein, beispielsweise durch einen Gewindeeingriff. Das Antriebsglied kann in einem Stück geformt sein, auch im Falle der bevorzugten Betätigbarkeit. Es kann alternativ aber auch aus separat gefertigten Teilen zusammengebaut sein, wobei die Teile vorzugsweise fest miteinander verbunden sind, so dass sie relativ zueinander nicht bewegt werden können.

Grundsätzlich kann das Antriebsglied aber auch aus mehreren separat gefertigten Teilen zusammengebaut sein und eines oder mehrere der Teile kann zu einem anderen der Teile beweglich sein, wobei auch bei solch einer Ausbildung des Antriebsglieds die mehreren Teile vorzugsweise axial relativ zueinander nicht beweglich sind. Andererseits entspricht es bevorzugten Ausführungen, wenn das Antriebsglied ein oder mehrere bewegbare Funktionselement(e) aufweist, wobei solch ein Funktionselement mit dem Antriebsglied oder einem Teil eines zusammengebauten Antriebsglieds in einem Stück geformt ist und die Beweglichkeit des betreffenden Funktionselements aufgrund einer Formelastizität erreicht wird, wofür sich insbesondere eine biegeelastische Gestaltung eignet. Die Ausführungen zur Formung in einem Stück und dem Zusammenbau aus separat geformten Teilen und auch die Ausführungen zu dem oder den Funktionselement(en) gelten in gleicher Weise auch für das Abtriebsglied. Ferner ist auch das Abtriebsglied bevorzugt nur translatorisch bewegbar. Grundsätzlich sind jedoch Ausführungen nicht ausgeschlossen, in denen das Abtriebsglied eine der Translation überlagerte Rotation ausführt.

Die erfindungsgemäße Vorrichtung eignet sich auch für eine automatisierte Verabreichung des Produkts. In solch einer Weiterbildung umfasst sie eine Antriebsfeder, die die Bewegung des Antriebsglieds in die Ausschüttposition vorzugsweise allein bewirkt oder zumindest unterstützt. Die Antriebsfeder ist so angeordnet, dass sie durch die Bewegung des Antriebsglieds in die Auslöseposition gespannt wird. Bei Auslösung setzt sie die durch das Spannen erzeugte Federenergie frei. Sie kann hierbei den Benutzer unterstützen, falls der Benutzer durch Ausübung eines axialen Drucks auf ein entsprechendes Betätigungsglied, das unmittelbar vom Antriebsglied gebildet werden kann, die Ausschüttung bewirkt. In bevorzugten Ausführungen bewirkt die Antriebsfeder wie gesagt alleine den Ausschüttvorgang nach Auslösung der Vorrichtung, vorzugsweise nach Auslösung des in der Auslöseposition befindlichen Antriebsglieds. Vorzugsweise wirkt die Antriebsfeder über das Antriebsglied auf das Abtriebsglied. Von Vorteil ist, wenn die Antriebsfeder direkt auf das Antriebsglied oder das Antriebsglied direkt auf das Abtriebsglied wirkt oder wirken. Das Wort "oder" wird hier wie auch stets sonst in seiner üblichen logischen Bedeutung als "inklusiv oder" verstanden, umfasst also die Bedeutung von "entweder...oder" und auch die Bedeutung von "und", soweit der jeweils konkrete Zusammenhang nicht ausschließlich nur eine eingeschränkte Bedeutung zulässt. In Bezug auf beispielsweise die Kraftübertragung bedeutet dies, dass in einer ersten Variante nur die Antriebsfeder direkt auf das Antriebsglied, in einer alternativen zweiten Variante nur das Antriebsglied direkt auf das Abtriebsglied und in noch einer alternativen Variante sowohl die Antriebsfeder direkt auf das Antriebsglied und das Antriebsglied direkt auf das Abtriebsglied wirkt, wobei solch einer zweimal direkten Kraftübertragung der Vorzug gegeben wird. Eine Automatisierung der Ausschüttung mittels Antriebsfeder hat den Vorteil, dass der Benutzer für die Ausschüttung keine Kraft ausüben muss oder in Ausführungen, in denen die Feder nur als Servofeder unterstützend wirkt, eine verringerte Kraft erforderlich ist. Wird das Ausschütten allein manuell bewirkt, besteht zum einen die Gefahr, dass im Falle einer Verabreichung mit infundierendem Teil der Ort der Verabreichung, d.h. die Eindringtiefe des infundierenden Teils, verändert wird. Ferner kann die Vorschubgeschwindigkeit des Kolbens bei Verwendung einer Antriebsfeder vergleichmäßigt, der Druck des austretenden Produktfluids also über die Zeit der jeweiligen Verabreichung verstetigt werden.

In Ausführungen, in denen das Antriebsglied nicht nur axial beweglich, sondern auch drehbeweglich ist, kann die Antriebsfeder beispielsweise eine Torsionsfeder sein. Sie kann in allen Ausführungen insbesondere axial auf das Antriebsglied wirken, gegebenenfalls zusätzlich auch in Umfangsrichtung um die Translationsachse. Bevorzugten Ausführungen entspricht es, wenn die Antriebsfeder nur axial auf das Antriebsglied wirkt. Die axial wirkende Feder kann funktional beispielsweise eine Zugfeder sein, bevorzugt ist sie als Druckfeder eingebaut. Sie wirkt vorzugsweise in Vortriebsrichtung auf das Antriebsglied, vorteilhafterweise direkt, indem sie an dem Antriebsglied abgestützt ist. Sie kann in die Gegenrichtung, vorzugsweise gegen die Vortriebsrichtung, am Gehäuse oder einer anderen Komponente der Vorrichtung abgestützt sein, wobei die andere Komponente mit dem Gehäuse in Richtung der abzustützenden Federkraft vorzugsweise unbeweglich verbunden ist. Handelt es sich wie bevorzugt um eine axiale Federkraft, ist die betreffende Komponente axial vorzugsweise unbeweglich, gegebenenfalls auch gänzlich unbeweglich mit dem Gehäuse verbunden. In bevorzugten Ausführungen ist die Antriebsfeder an dem Dosierglied abgestützt. Umgeben das Antriebsglied und das Dosierglied einander, wobei vorzugsweise das Dosierglied das Antriebsglied umgibt, so kann die Antriebsfeder vorteilhafterweise in einem zwischen dem Antriebsglied und dem Dosierglied verbleibenden Ringspalt angeordnet sein, zumindest teilweise.

Die flexible Kopplung von Antriebsglied und Abtriebsglied umfasst in bevorzugten Ausführungen eine Ausgleichsfeder, die bei der Bewegung des Antriebsglieds in die Auslöseposition auf das Abtriebsglied eine Federkraft ausübt, die den Dosierhub des Abtriebsglieds bewirkt. Die Ausgleichsfeder kann beispielsweise funktional als Torsionsfeder eingebaut sein, durch die Bewegung des Antriebsglieds in die Auslöseposition also auf Torsion beansprucht werden, falls das Antriebsglied und das Abtriebsglied beispielsweise mittels eines Schraubgelenks gekoppelt sind. Bevorzugter sind das Antriebsglied und das Abtriebsglied jedoch nur axial geradgeführt ohne relative Zwangsdrehung. Dass eine relative Zwangsdrehung, wie sie beispielsweise ein Schraubgelenk verwirklicht, nicht vorgesehen ist, bedeutet allerdings nicht, dass das Antriebsglied und das Abtriebsglied relativ zueinander nicht drehbar sein dürfen, sondern bedeutet nur, dass eine Zwangsdrehbewegung nicht erforderlich ist, um mittels des Hubs des Antriebsglieds den jeweils zugeordneten Hub des Abtriebsglieds zu erzeugen. Das Antriebsglied und das Abtriebsglied können so miteinander gekoppelt sein, dass sie relativ zueinander axial beweglich und auch um die Translationsachse relativ zueinander drehbar sind, wie gesagt jedoch bevorzugt nicht über ein Gelenk, das bei einer relativen Axialbewegung eine relative Drehbewegung erzwingt. In bevorzugten Ausführungen sind das Abtriebsglied und das Antriebsglied relativ zueinander axial linear bewegbar, relativ zueinander aber um die Translationsachse nicht verdrehbar. Sie können in diesen Ausführungen aneinander oder an einer anderen oder jeweils an einer anderen Komponente der Vorrichtung verdrehgesichert lineargeführt sein. So kann das Abtriebsglied beispielsweise an der Kolbenstange oder das Antriebsglied beispielsweise an dem Gehäuse linear und vorzugsweise verdrehgesichert geführt sein.

Vorzugsweise wirkt die Ausgleichsfeder auf das Antriebsglied oder das Abtriebsglied in axialer Richtung, besonders bevorzugt nur in axialer Richtung. Sie kann funktional insbesondere eine Druckfeder sein, grundsätzlich kann sie aber auch als Zugfeder wirken. Die Ausgleichsfeder kann über ein oder mehrere Zwischenglieder oder vorzugsweise direkt auf das Antriebsglied oder das Abtriebsglied wirken, im bevorzugten letzteren Fall, indem sie sich unmittelbar an dem Antriebsglied oder dem Abtriebsglied abstützt.

Die Kopplung von Abtriebsglied und Kolbenstange kann mit Vorteil als einfacher Mitnahmeeingriff gebildet sein, so dass das Abtriebsglied bei seiner Bewegung in die Vortriebsrichtung die Kolbenstange 1:1 mitnimmt oder von der Kolbenstange mitgenommen wird. Vorzugsweise nimmt das Abtriebsglied die Kolbenstange mit, d. h. der Kraftfluss verläuft vom Antriebsglied über das Abtriebsglied auf die Kolbenstange.

In bevorzugten Ausführungen ist die Kolbenstange eine Zahnstange mit wenigstens einer Zahnreihe von axial hintereinander angeordneten Zähnen. Die Zähne sind so geformt, dass ein in die Zahnreihe eingreifender Mitnehmer, den vorzugsweise das Abtriebsglied bildet, bei der Bewegung in die Vortriebsrichtung auf eine Rückflanke eines der Zähne der Zahnreihe und dadurch die Kolbenstange in die Vortriebsrichtung drückt. Die Zähne der Zahnreihe sind vorteilhafterweise so geformt, dass sie jeweils an ihrer Vorderflanke eine geringere Neigung zur Vortriebsrichtung aufweisen als an ihrer Rückflanke. Die Rückflanken können insbesondere senkrecht zur Vortriebsrichtung weisen. Bei Ausbildung der Kolbenstange als Zahnstange weist die Vorrichtung ferner eine Rückzugssicherung auf, die ebenfalls im Eingriff mit der Zahnreihe oder einer anderen Zahnreihe der Kolbenstange ist und in diesem Rückhalteeingriff verhindert, dass sich die Kolbenstange bei dem Dosierhub des Abtriebsglieds gegen die Vortriebsrichtung bewegen kann. Vorzugsweise weist die Kolbenstange wenigstens zwei der beschriebenen Zahnreihen auf, und das Abtriebsglied und vorzugsweise auch die Rückzugssicherung greifen in jede dieser Zahnreihen ein.

Das Dosierglied gibt in den Auswahlpositionen den Dosierhub des Abtriebsglieds vorzugsweise durch Anschlagkontakt vor, begrenzt also den variablen Dosierhub jeweils durch einen Anschlagkontakt. Das Dosierglied ist relativ zu dem Gehäuse vorzugsweise um die Translationsachse der Kolbenstange drehbar, so dass die Auswahlpositionen unterschiedliche Drehwinkelpositionen sind. Die Auswahlpositionen können insbesondere diskret vorgegebene Rastpositionen sein. Vorzugsweise bildet das Dosierglied oder das Abtriebsglied unmittelbar selbst einen Anschlag, besonders bevorzugt bildet sowohl das Dosierglied als auch das Antriebsglied jeweils einen der Anschläge, die zur Begrenzung des Dosierhubs miteinander in den Anschlagkontakt gelangen. Das Dosierglied kann insbesondere als Treppenglied in der Art des Dosierglieds der US 6,277,101 B1 gebildet sein, die diesbezüglich in Bezug genommen wird. Alternativ kann das Dosierglied den Dosieranschlag für die jeweilige Auswahlposition auch als einen spiralig kontinuierlich umlaufenden Anschlag bilden. Ein Beispiel für solch ein Dosierglied wird in der US 6,699,224 B2 offenbart, die diesbezüglich in Bezug genommen wird.

Zur Ausgestaltung der Fördereinrichtung wird ergänzend die internationale Anmeldung der Anmelderin vom heutigen Tage mit dem Titel "Vorrichtung zur Verabreichung eines injizierbaren Produkts mit Längenausgleich" in Bezug genommen, insbesondere deren Ansprüche.

Vorteilhafte Merkmale werden auch in den Unteransprüchen und deren Kombinationen beschrieben.

Nachfolgend werden Ausführungsbeispiele der Erfindung anhand von Figuren erläutert. An den Ausführungsbeispielen offenbar werdende Merkmale bilden jeweils einzeln und in jeder Merkmalskombination die Gegenstände der Ansprüche und auch die vorstehend beschriebenen Ausführungsformen vorteilhaft weiter. Es zeigen:
- Figur 1: ein erstes Ausführungsbeispiel einer erfindungsgemäßen Vorrichtung in einem Auswahlzustand,
- Figur 2: die Vorrichtung der Figur 1 in einem Auslösezustand,
- Figur 3: die Vorrichtung im Zustand der Figur 1 in einer Ansicht,
- Figur 4: die Vorrichtung im Zustand der Figur 2 in einer Ansicht,
- Figur 5: ein zweites Ausführungsbeispiel einer erfindungsgemäßen Vorrichtung in einem Auswahlzustand
- Figur 6: die Vorrichtung der Figur 5 in einem Auslösezustand,
- Figur 7: die Vorrichtung der Figur 5 in einem anderen Längsschnitt,
- Figur 8: die Vorrichtung der Figur 5 in einem Querschnitt,
- Figur 9: die Vorrichtung der Figur 5 im Auswahlzustand in einer Seitenansicht,
- Figur 10: die Vorrichtung der Figur 5 im Auslösezustand in der Seitenansicht,
- Figur 11: ein erstes Ausführungsbeispiel eines Ausschüttungszählers vor einer ersten Ausschüttung,
- Figur 12: den Ausschüttungszähler der Figur 11 nach einer ersten Ausschüttung,
- Figur 13: die Vorrichtung der Figuren 5-12 in einem Querschnitt mit dem Ausschüttungszähler,
- Figur 14: die Vorrichtung der Figur 5 vor der ersten Ausschüttung mit dem Ausschüttungszähler der Figur 11 und einer zusätzlichen Restmengenanzeige,
- Figur 15: die Vorrichtung der Figur 14 nach der ersten Ausschüttung,
- Figur 16: eine vom ersten Ausführungsbeispiel abgeleitete Vorrichtung in einem Auswahlzustand mit einem Ausschüttungszähler eines zweiten Ausführungsbeispiels,
- Figur 17: die Vorrichtung der Figur 16 nach mehreren Ausschüttungen in einer Seitenansicht auf eine Anzeige des Ausschüttungszählers,
- Figur 18: den Ausschüttungszähler der Figur 16 in einem ersten Zustand,
- Figur 19: den Ausschüttungszähler der Figur 16 in einem zweiten Zustand und
- Figur 20: eine erfindungsgemäße Vorrichtung mit einem Ausschüttungszähler eines dritten Ausführungsbeispiels.

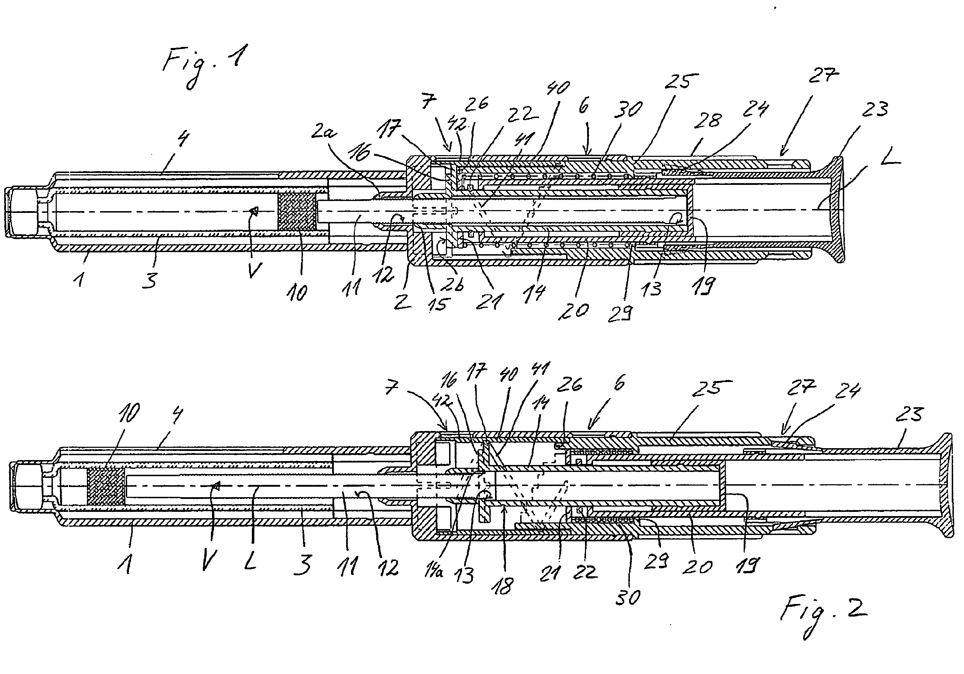

Figur 1 zeigt in einem Längsschnitt eine Vorrichtung für die Verabreichung eines injizierbaren Produkts nach einem ersten Ausführungsbeispiel. Bei der Vorrichtung handelt es sich um ein Injektionsgerät in der Form eines Injektionspen. Die Vorrichtung umfasst ein Gehäuse mit einem distalen Gehäuseteil 1 und einem proximalen Gehäuseteil 2, die miteinander unlösbar verbunden sind, alternativ auch lösbar miteinander verbunden sein können. Das Gehäuseteil 1 bildet eine Aufnahme für ein mit dem zu injizierenden Produkt gefülltes Behältnis 3. Bei dem Behältnis 3 handelt es sich um eine vorabgefüllte Ampulle. Das Behältnis 3 weist an einem distalen Ende einen Auslass auf und wird an einem proximalen Ende fluiddicht von einem Kolben 10 verschlossen. Der Kolben 10 ist in dem Behältnis 3 in eine Vortriebsrichtung V auf den Auslass zu axial bewegbar, um Produkt auszuschütten.

In Figur 1 befindet sich die Vorrichtung in einem Auswahlzustand, in dem die zu verabreichende Produktdosis eingestellt wird.

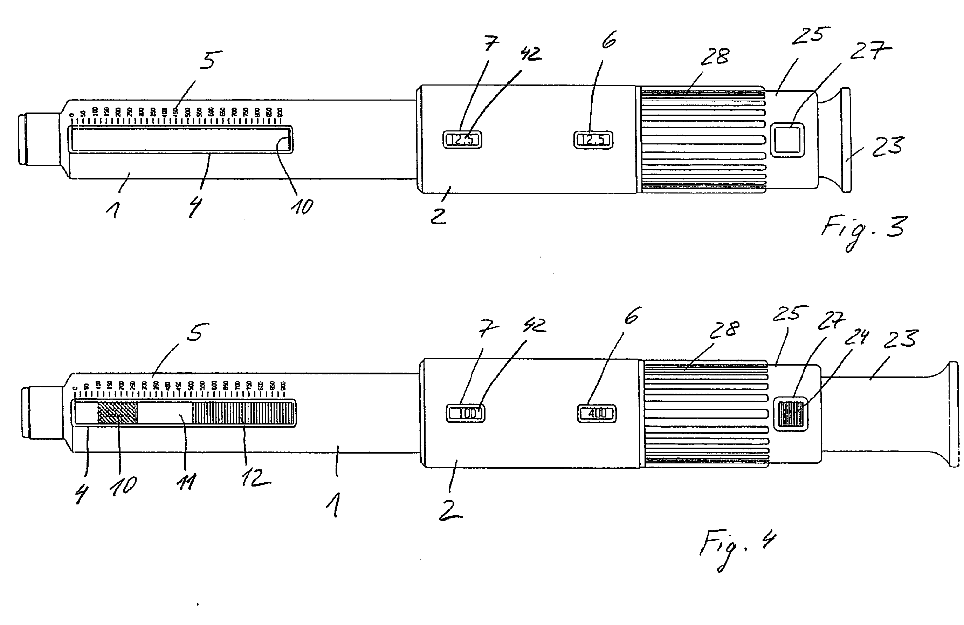

Figur 3 zeigt die Vorrichtung in einer Seitenansicht ebenfalls im Auswahlzustand. Das Gehäuseteil 1 ist beispielhaft über den größten Teil seiner axialen Länge als kreiszylindrische Hülse geformt. Es weist ein langgestrecktes Sichtfenster 4 auf, das die Sicht auf das durchsichtige Behältnis 3 freigibt. Das Sichtfenster 4 ist so lang, dass der Kolben 10 über seine gesamte Wegstrecke, die er im Behältnis 3 zurücklegen kann, sichtbar ist. Auf dem Umfang des Gehäuseteils 1 erstreckt sich neben dem Sichtfenster 4 axial eine Dosisskala 5. Der Kolben 10 dient als Marker, im Ausführungsbeispiel eine distale Kante des Kolbens 10, so dass anhand der axialen Position des Kolbens 10 auf der Dosisskala 5 die noch im Behältnis 3 befindliche Produktmenge, die Restmenge, abgelesen werden kann. Die Vorrichtung weist zwei weitere Anzeigen auf, eine Dosisanzeige 6 und eine weitere Restmengenanzeige 7. Die Dosisanzeige 6 zeigt die eingestellte Dosis, und die Restmengenanzeige 7 zeigt nochmals die verfügbare Restmenge an. Die Anzeigen 6 und 7 sind jeweils durch ein im Gehäuseteil 2 ausgenommenes Sichtfenster und jeweils eine Dosisskala gebildet, wobei unterschiedliche Komponenten der Vorrichtung als Träger der Dosisskalen dienen. Die Dosisskala der Dosisanzeige 6 ist an einem Umfang eines drehbeweglich mit dem Gehäuseteil 2 verbundenen Dosierglieds 25 angebracht. Der Benutzer kann mittels einer Dosierdrehbewegung des Dosierglieds 25 die gewünschte Produktdosis einstellen. Im Ausgangszustand der Vorrichtung zeigt die Dosisanzeige 6 eine Minimaldosis an, im Ausführungsbeispiel "12.5"; die Minimaldosis kann aber auch die Nulldosis sein.

Figur 2 zeigt die Vorrichtung nach dem Einstellen der Produktdosis in einem Auslösezustand. Die im Auslösezustand befindliche Vorrichtung ist ferner in einer Seitenansicht in Figur 4 dargestellt.

Die Vorrichtung umfasst eine Kolbenstange 11, die relativ zu dem Behältnis 3 in die Vortriebsrichtung V bewegbar ist und bei dieser Bewegung auf den Kolben 10 wirkt, um diesen im Behältnis 3 vorzutreiben. Die Kolbenstange 11 ist gegen eine Bewegung entgegen der Vortriebsrichtung V gesichert, kann translatorisch also nur in die Vortriebsrichtung V bewegt werden. Sie ist als Zahnstange gebildet mit zwei an gegenüberliegenden Längsseiten geformten Zahnreihen 12 aus Sägezähnen, in die zur Verhinderung einer Bewegung entgegen der Vortriebsrichtung V eine Rückzugssicherung 2a eingreift. Die Rückzugssicherung 2a ist in einem Stück mit dem Gehäuse, hier beispielhaft in einem Stück mit dem Gehäuseteil 2 geformt, könnte alternativ aber auch separat geformt und axial fest mit dem Gehäuse 1, 2 verbunden sein.

Die Vorrichtung umfasst ferner ein erstes Förderglied 14, im Folgenden Abtriebsglied 14, und ein zweites Förderglied 20, im Folgenden Antriebsglied 20, die zusammen mit der Kolbenstange 11, dem Kolben 10 und Kopplungen eine Fördereinrichtung der Vorrichtung bilden. Das Abtriebsglied 14 ist mit der Kolbenstange 11 in einem Zahneingriff und weist hierfür Mitnehmer 15 auf, die bevorzugt, wie im Ausführungsbeispiel, an dem distalen Ende des Abtriebsglieds 14 geformt sind. Der Zahneingriff ist ein Mitnahmeeingriff, in dem das Abtriebsglied bei einer Bewegung in die Vortriebsrichtung V die Kolbenstange 11 mitnimmt. Der Mitnahmeeingriff ist ferner so gestaltet, dass das Abtriebsglied 14 relativ zu der Kolbenstange 11 gegen die Vortriebsrichtung V bewegbar ist, um die zu verabreichende Produktdosis einstellen zu können. Zum Lösen des Mitnahmeeingriffs sind die Mitnehmer 15 elastisch aus dem Zahneingriff mit der jeweils zugeordneten Zahnreihe 12 nach außen biegbar, so dass sie bei der Bewegung gegen die Vortriebsrichtung V über die Zahnreihen 12 gleiten können, während die Kolbenstange 11 im Rückhalteeingriff mit der Rückzugssicherung 2a in ihrer axialen Position gehalten wird.

Das Antriebsglied 20 ist zwischen einer im Auswahlzustand der Figur 1 eingenommenen Ausschüttposition und einer in Figur 2 eingenommenen Auslöseposition axial hin und her bewegbar. Die Ausschüttposition wird durch Anschlagkontakt, im Ausführungsbeispiel ein Anschlagkontakt in axialer Richtung, vorgegeben. Ein die Ausschüttposition bestimmender Ausschüttanschlag 2b wird vom Gehäuseteil 2 gebildet. Das Antriebsglied 20 weist als Gegenstück an einem distalen Ende einen Anschlag 21 auf, der aber erst über einen Flansch 16 des Abtriebsglieds 14 und somit indirekt in der Ausschüttposition gegen den Ausschüttanschlag 2b drückt. Der Flansch 16 ragt von einem Hülsenabschnitt des Abtriebsglieds 14 radial nach außen ab.

Die Auslöseposition wird durch einen Halteeingriff vorgegeben, in den ein Eingriffselement 24 des Antriebsglieds 20 gelangt, wenn es gegen die Vortriebsrichtung V bis in die in Figur 2 dargestellte Auslöseposition bewegt wird. In dem Halteeingriff ist das Eingriffselement 24 in die Vortriebsrichtung V durch Anschlagkontakt relativ zu dem Gehäuse 1, 2 festgelegt. Der axiale Hub des Antriebsglieds 20 aus der Ausschüttposition in die Auslöseposition ist ungeachtet der eingestellten Produktdosis stets gleich lang.

Die Produktdosis ist mittels des Dosierglieds 25 einstellbar. Das Dosierglied 25 ist um die Längsachse L drehbar mit dem Gehäuseteil 2 verbunden, relativ zum Gehäuseteil 2 axial jedoch nicht beweglich. Das Dosierglied 2 weist einen von außen zugänglichen Hülsenabschnitt mit einer äußeren Profilierung 28 auf, den der Benutzer für die Einstellung der Dosis greifen kann. Das Dosierglied 25 weist an einem distalen Ende, innerhalb des Gehäuseteils 2, um die Längsachse L verteilt angeordnete Dosieranschläge 26 auf, die von einer distalen Stirnfläche des Dosierglieds 25 gebildet werden, alternativ aber beispielsweise auch weiter proximal von der Umfangsfläche des Dosierglieds 25 abragen könnten. Die Dosieranschläge 26 laufen im Groben gesehen wie eine Spirale um die Längsachse L um, bilden jedoch in Umfangsrichtung Treppenstufen, wie insbesondere in Figur 2 zu erkennen ist, in der die Treppenstufen, d. h. die einzelnen Dosieranschläge 26, strichliert eingezeichnet sind. Die auf diese Weise diskret gebildeten Dosieranschläge 26 befinden sich entsprechend der Feinheit der Treppenstufung auf unterschiedlichen axialen Höhen. Das Dosierglied 25 ist mittels der Dosierdrehbewegung in unterschiedliche Auswahlpositionen verstellbar, eine Auswahlposition pro Dosieranschlag 26. In den einzelnen Auswahlpositionen liegen sich der Dosieranschlag 26, der der jeweiligen Auswahlposition zugeordnet ist, und ein von dem Abtriebsglied 14 gebildeter Gegenanschlag 17 axial zugewandt gegenüber. Der Gegenanschlag 17 ist an dem Flansch 16 des Abtriebsglieds 14 geformt und verlängert diesen in radialer Richtung und in Umfangsrichtung über einen im Vergleich zu einem vollen Umlauf um die Längsachse L kleinen Bogenwinkel. Durch Drehen des Dosierglieds 25 kann sukzessive jeder der Dosieranschläge 26 in die dem Gegenanschlag 17 axial gegenüberliegende Drehwinkelposition bewegt werden. Der axiale Abstand zwischen dem der jeweiligen Auswahlposition zugeordneten Dosieranschlag 26 und dem Gegenanschlag 17 bestimmt die Produktdosis, allerdings nur, solange die im Behältnis 3 befindliche Produktmenge, die Restmenge, wenigstens so groß wie die eingestellte Produktdosis ist.

Die Vorrichtung weist eine flexible Kopplung auf, die das Abtriebsglied 14 mit dem Antriebsglied 20 koppelt. Die Flexibilität besteht darin, dass die Kopplung eine Relativbewegung zwischen dem Antriebsglied 20 und dem Abtriebsglied 14 über zumindest einen Teil des axialen Hubs des Antriebsglieds 20 in jeder der Auswahlpositionen des Dosierglieds 25 zulässt. Dennoch sorgt die Kopplung dafür, dass der Hub des Antriebsglieds 20 in die Auslöseposition einen Dosierhub des Abtriebsglieds 14 und der Hub des Antriebsglieds 20 in die Ausschüttposition einen Ausschütthub des Abtriebsglieds 14 bewirkt. Die axiale Länge des Dosierhubs entspricht der axialen Länge des Ausschütthubs, wobei diese Länge variabel ist und von der eingestellten Produktdosis, d. h. von der Auswahlposition des Dosierglieds 25 abhängt, während die axiale Länge des Hubs des Antriebsglieds 20 aber dennoch stets die gleiche ist. Der Dosierhub ist die Hubbewegung des Abtriebsglieds 14 gegen die Vortriebsrichtung V aus der in Figur 1 eingenommenen distalen Axialposition bis gegen den der eingestellten Auswahlposition des Dosierglieds 25 zugeordneten Dosieranschlag 26, und der Ausschütthub ist die Hubbewegung des Abtriebsglieds 14 in die Gegenrichtung bis gegen den Ausschüttanschlag 2b.

Die Kopplung umfasst ein Kupplungselement 18 (Figur 2) und ein Kupplungsgegenelement 22, die in der Ausschüttposition des Antriebsglieds (20) miteinander in einem Kupplungseingriff sind. Der Kupplungseingriff ist ein Mitnahmeeingriff, der bei einer axialen Bewegung des Antriebsglieds 20 in Richtung auf die Auslöseposition, den Dosierhub des Abtriebsglieds 14 bewirkt und sich am Ende des Dosierhubs automatisch löst, wenn das Antriebsglied 20 weiter in Richtung auf die Auslöseposition bewegt wird, so dass das Antriebsglied 20 ein erstes Stück seines Hubs gemeinsam mit dem Abtriebsglied 14 und ein zweites Stück des Hubs ohne das Abtriebsglied 14 zurücklegt. Im Ausführungsbeispiel ist das Kupplungselement 18 unmittelbar am Abtriebsglied 14 und das Kupplungsgegenelement 22 unmittelbar am Antriebsglied 20 geformt. In Abwandlungen könnte entweder das Kupplungselement 18 an einem Zwischenglied oder das Kupplungsgegenelement 22 an einem Zwischenglied oder jedes dieser Elemente 18 und 22 an jeweils einem anderen Zwischenglied geformt sein, solange nur die beschriebene Funktion der flexiblen Kopplung erfüllt wird. Eine Kopplung durch unmittelbaren Eingriff von Antriebsglied 14 und Abtriebsglied 20 wird jedoch im Hinblick auf die Einfachheit der Konstruktion und insbesondere die Gewährleistung der ordnungsgemäßen Funktion der Kopplung bevorzugt Das Kupplungselement 18 ist als radiale Vertiefung, gegebenenfalls eine einzige Vertiefung, oder bevorzugt wie im Ausführungsbeispiel als umlaufende Nut oder in Form mehrerer diskreter Vertiefungen geformt. Das Kupplungsgegenelement 22 ist ein in radialer Richtung bezüglich der Längsachse L aus dem Kupplungseingriff elastisch abbiegbarer Schnapper mit einem Eingriffsnocken am Ende. Im Ausführungsbeispiel sind zwei Kupplungsgegenelemente 22 vorgesehen, die um die Längsachse L um einen Winkel von 180° zueinander versetzt sind. Die Anordnung von Vertiefung und elastischem Schnapper kann grundsätzlich auch umgekehrt werden, d. h. die Vertiefung könnte am Antriebsglied 20 und der elastische Schnapper am Abtriebsglied 14 vorgesehen sein. Das Kupplungsgegenelement 22 wird am Ende des Dosierhubs im Falle einer weiterhin auf das Antriebsglied 20 gegen die Vortriebsrichtung V ausgeübten Kraft gelöst, indem das Kupplungsgegenelement 22 durch elastisches Abbiegen aus dem Kupplungseingriff nachgibt.

Die lösbare Kupplung ist im Ausführungsbeispiel mit zwei Kupplungsgegenelementen 22 gebildet, grundsätzlich würde auch ein einziges Kupplungsgegenelement 22 genügen. Alternativ können um die Längsachse L verteilt auch drei oder noch mehr Kupplungsgegenelemente 22 vorgesehen sein und beispielsweise mit dem gleichen Kupplungselement 18 zusammenwirken. Die Kupplungsgegenelemente 22 sind im Ausführungsbeispiel als elastische Schnapper gebildet. Alternativ könnte beispielsweise ein elastisch in seinem Querschnitt nachgiebiger oder elastisch aufweitbarer Ring, der im Kupplungseingriff das als Vertiefung gebildete Kupplungselement 18 umspannt, das einzige Kupplungsgegenelement 22 bilden. Die Verhältnisse hinsichtlich des Kupplungselements 18 und des oder der Kupplungselements(e) 22 können auch umgekehrt werden, indem das Kupplungselement 18 durch einen oder mehrere Schnapper in der Art der Kupplungsgegenelemente 22 oder durch den erwähnten Ring und die Kupplungsgegenelemente 22 durch eine Vertiefung in der Art des Kupplungselements 18 ersetzt wird bzw. werden.

Der Kupplungseingriff wird nicht nur dann gelöst, wenn der Dosierhub vollständig ausgeführt wird, das Abtriebsglied 14 also in Anschlagkontakt mit dem der Auswahlposition des Dosierglieds 25 entsprechenden Dosieranschlag 26 gelangt, sondern auch dann, wenn die Hubbewegung des Abtriebsglieds 14 anderweitig durch einen Widerstand beendet wird, der die Haltekraft des Kupplungseingriffs übersteigt. Dies ist dann der Fall, wenn die im Behältnis 3 noch verfügbare Restmenge kleiner ist als die der eingestellten Auswahlposition des Dosierglieds 25 entsprechende Produktdosis. Dieser Zustand ist in Figur 2 dargestellt. Der Dosierhub des Abtriebsglieds 14 wird dann vor Erreichen des Dosieranschlags 26 beendet, wenn die Kolbenstange 11 in einen axialen Anschlagkontakt mit dem Abtriebsglied 14 gelangt. Hierfür weist die Kolbenstange 11 an ihrem proximalen Ende einen Restmengen-Anschlag 13 und das Abtriebsglied 14 in seinem distalen Endbereich einen Restmengen-Gegenanschlag 14a auf.

Um die Ausschüttung der eingestellten Produktdosis zu automatisierten, ist die Vorrichtung mit einer Antriebsfeder 30 ausgestattet. Die Antriebsfeder 30 wirkt in die Vortriebsrichtung V auf das Antriebsglied 20 und stützt sich gegen die Vortriebsrichtung V an dem Dosierglied 25 und somit letztlich am Gehäuseteil 2 ab. Sie wird durch die Bewegung des Antriebsglieds 20 in die Auslöseposition gespannt und bewirkt bei einem Auslösen der Vorrichtung die Bewegung des Antriebsglieds 20 in die Ausschüttposition, d. h. in die Vortriebsrichtung V. Für die Abstützung weist das Antriebsglied 20 an einem distalen Ende eine im Bereich des Anschlags 21 geformte, mit gleichem Bezugszeichen versehene Schulter 21 und das Dosierglied 25 in einem axialen Abstand eine Schulter 29 auf. Die Antriebsfeder 30 ist über einen größeren Teil ihrer axialen Länge in einem Ringspalt aufgenommen, der zwischen dem Antriebsglied 20 und einem distalen Hülsenabschnitt des Dosierglieds 25 verbleibt. Sie wird auf Druck beansprucht, ist funktional also eine Druckfeder, und als Schraubenfeder geformt.

Das Abtriebsglied 14 ist aus zwei Teilen gefüge, dem genannten Hülsenabschnitt, der das Kupplungselement 18 bildet und von dem die Mitnehmer 14 und der Flansch 16 abragen, und einem Endteil 19, das in der Art einer Kappe am proximalen Ende des Hülsenabschnitts des Antriebsglieds 14 angeordnet und mit dem Hülsenabschnitt fest verbunden ist. Der Hülsenabschnitt und das Endteil 19 können als ein einziges Teil angesehen werden.

Das Antriebsglied 20 ist ebenfalls aus zwei separat gefertigten Teilen zusammengesetzt, die im zusammengesetzten Zustand als ein einziges Teil betrachtet werden können. Bei den beiden Teilen handelt es sich um einen distalen Hülsenabschnitt, der das Abtriebsglied 14 umgibt und insbesondere die Kupplungsgegenelemente 22 sowie die Schulter 21 bildet, an der sich die Antriebsfeder 30 in die Vortriebsrichtung V abstützt, und um einen proximalen Hülsenabschnitt, der aus dem Dosierglied 25 gegen die Vortriebsrichtung V herausragt und an seinem Ende ein Betätigungsteil 23 aufweist, an dem der Benutzer das Antriebsglied 20 greifen und gegen die Vortriebsrichtung V in die Auslöseposition ziehen kann.

Im Bereich des Betätigungsteils 23 ist das Eingriffselement 24 geformt. Das Eingriffselement 24 ist ein biegeelastischer Schnapper, der in einer Ausnehmung des hülsenformigen Betätigungsteils 23 geformt ist und elastisch nach radial außen vorgespannt an einer Mantelinnenfläche des Dosierglieds 25 anliegt, solange sich das Antriebsglied 20 noch nicht in der Auslöseposition befindet. Sobald die Auslöseposition erreicht wird, schnappt das Eingriffselement 24 in einen Durchbruch vor, der im Dosierglied 25 geformt ist und ein Eingriffsgegenelement 27 für den Halteeingriff des Antriebsglieds 20 bildet. Im Ausführungsbeispiel weisen das Antriebsglied 20 in Umfangsrichtung um die Längsachse L zwei um 180° zueinander versetzte Eingriffselemente 24 und das Dosierglied 25 entsprechend zwei Eingriffsgegenelemente 27 jeweils der genannten Art auf.

Die Vorrichtung verfügt wie bereits erwähnt über eine Dosisanzeige, bei 6, zum Anzeigen der eingestellten Produktdosis, eine erste Restmengenanzeige, bei 7, zum Anzeigen der mit dem nächsten Ausschütthub ausschüttbaren Produktmenge und die bereits genannte Restmengenanzeige, bei 4, die eine zweite Restmengenanzeige 4, 5, 10 bildet und die im Behältnis 3 insgesamt noch verfügbare Produktmenge anzeigt. Die Dosisanzeige wird von dem Dosierglied 25 und dem Gehäuseteil 2 gebildet, indem das Dosierglied 25 in einem in das Gehäuseteil 2 hineinragenden Hülsenabschnitt am äußeren Umfang eine Dosisskala mit in Umfangsrichtung hintereinander angeordneten Dosiswerten aufweist und das Gehäuseteil 2 axial auf der Höhe dieser Dosisskala mit dem Sichtfenster 6 versehen ist. Durch das Sichtfenster 6 ist in den Auswahlpositionen des Dosierglieds 25 der jeweils der Auswahlposition zugeordnete Dosiswert der Dosisskala erkennbar.

Die erste Restmengenanzeige, bei 7, umfasst ein erstes Restmengenglied 40 und das Gehäuseteil 2 als zweites Restmengenglied. Das erste Restmengenglied 40 weist an einer äußeren Umfangsfläche, die einer inneren Umfangsfläche des Gehäuseteils 2 zugewandt ist, eine um die Längsachse L erstreckte Restmengen-Dosisskala 42 aus Dosiswerten auf. Die Dosisskala 42 der Restmengenanzeige kann der Dosisskala des Dosierglieds 25 entsprechen. Die Dosisskala 42 der Restmengenanzeige kann durch das Sichtfenster 7 des zweiten Restmengenglieds, des Gehäuseteils 2, abgelesen werden. Das Sichtfenster bildet somit einen Marker der Restmengenanzeige 7, 42.

Das erste Restmengenglied 40 ist mit dem Abtriebsglied 14 gekoppelt. Im Ausführungsbeispiel sind das Restmengenglied 40 und das Abtriebsglied 14 miteinander in einem Führungseingriff. Sie bilden miteinander ein Kurvengetriebe. Im Führungseingriff wird der Dosierhub des Abtriebsglieds 14 in eine Drehhubbewegung des ersten Restmengenglieds 40 um die Längsachse L und der Ausschütthub wird in eine Rückdrehbewegung des ersten Restmengenglieds 40 umgewandelt. Das erste Restmengenglied 40 weist an einer dem Abtriebsglied 14 zugewandten inneren Umfangsfläche eine Führungskurve 41 auf, die um die Längsachse L eine Schraubenlinie bildet. Das Abtriebsglied 14 bildet mit seinem Dosier-Gegenanschlag 17 ein Eingriffselement, das mit der Führungskurve 41 in dem Führungseingriff des Kurvengetriebes ist. Das Restmengenglied 40 ist getriebetechnisch gesehen das Führungsglied, und das Abtriebsglied 14 ist das Eingriffsglied des Kurvengetriebes.

Das Kurvengetriebe ist als Schraubgelenk mit der Längsachse L als Schraubachse ausgeführt. Die Steigung der Führungskurve 41 ist so groß, dass im Führungseingriff 17, 41 bei den axialen Hubbewegungen des Abtriebsglieds 14 keine Selbsthemmung auftreten kann, sondern das erste Restmengenglied 40 um die Längsachse L entsprechend der Richtung der Hubbewegung des Abtriebglieds 14 frei hin und her dreht und sich die Dosiskala 42 der Restmengenanzeige 7, 42 unter dem Sichtfenster 7 vorbeibewegt.

Wie die Figuren 3 und 4 zeigen, können die eingestellte Produktdosis bei 6 und die tatsächlich mit dem nächsten Ausschütthub ausschüttbare Produktmenge, bei 7, auf einen Blick nebeneinander abgelesen werden. Aufgrund der Anordnung der jeweiligen Dosisskala am beweglichen Glied der jeweiligen Anzeige wird die Handhabung der Vorrichtung bei der Einstellung der Dosis und dem Ablesen vereinfacht und die Einstellung und Verabreichung der korrekten Dosis mit höherer Zuverlässigkeit gewährleistet. Der Benutzer muss lediglich das Gehäuse 1, 2 mit der einen Hand greifen und kann dann bequem mit der anderen Hand die Dosis einstellen und bei 6 und 7 die entsprechenden Informationen - eingestellte Dosis und ausschüttbare Dosis - ablesen. Von Vorteil ist ferner, dass auch die zweite Restmengenanzeige 4, 5, 10 in der axialen Flucht oder zumindest im Wesentlichen in der axialen Flucht zu den beiden Anzeigen bei 6 und 7 angeordnet ist, so dass alle drei Anzeigen ohne die Vorrichtung drehen zu müssen auf einen Blick nebeneinander abgelesen werden können.

Ein weiterer Vorteil der beiden voneinander unabhängigen Dosisskalen, nämlich der Dosisanzeige bei 6 für die eingestellte Produktdosis und der ersten Restmengenanzeige bei 7, ist die einfache und sichere Kontrollmöglichkeit. Solange die im Behältnis 3 noch fügbare Restmenge wenigstens so groß wie die eingestellte Produktdosis ist, zeigen die beiden Anzeigen den gleichen Wert an. Der Benutzer erfährt auf einfache, unmissverständliche Art und Weise, dass mit dem nächsten Ausschütthub tatsächlich die eingestellte Produktdosis ausgeschüttet wird. Insbesondere sind keine weitergehenden Überlegungen und Merkvorgänge diesbezüglich erforderlich. Das gleiche gilt, wenn die Restmenge kleiner als die eingestellte Produktdosis ist.

Noch ein weiterer Vorteil liegt darin begründet, dass die Hubbewegung des Antriebsglieds 20 für alle einstellbaren Dosen stets gleichlang ist und der Benutzer daher noch zusätzlich die Gewähr hat, dass er sein Gerät korrekt gehandhabt hat, wenn das Betätigungsteil 23 des Antriebsglieds 20 die Auslöseposition einnimmt, auch wenn die Dosiswerte der Dosisanzeige bei 6 und der Restmengenanzeige bei 7 differieren.

Dic Antricbsfcdcr 30 ist ebenfalls ein Sichcrhcitsfaktor, insbesondere in Kombination mit der ersten Restmengenanzeige bei 7. Vielen Benutzern fällt es bereits schwer, den für die Ausschüttung erforderlichen Druck bei manuell durchzuführender Ausschüttung bis zum vollständigen Ausführen des Ausschütthubs auszuüben. Dies besorgt vorliegend jedoch die Antriebsfeder 30. Nach dem Auslösen kann der Benutzer ferner den mittels der Antriebsfeder 30 automatisierten Ausschüttvorgang durch Beobachten der ersten Restmengenanzeige kontrollieren, da die erste Restmengenanzeige 7, 42 aufgrund der Kopplung des ersten Restmengenglieds 40 mit dem Abtriebsglied 14 bei der Ausschüttung automatisch zurückgesetzt wird.

Die zweite Restmengenanzeige, bei 5, bietet noch eine zusätzliche Kontrollmöglichkeit zur Überprüfung der Korrektheit der ersten Restmengenanzeige 7, 42.

Nachfolgend wird die Handhabung der Vorrichtung anhand der Figuren 1 und 2, ferner unter Verweis auf die Figuren 3 und 4 beschrieben:
Der Benutzer erhält die Vorrichtung im Auswahlzustand, wie ihn Figur 1 darstellt. Das Behältnis 3 ist mit dem Produkt gefüllt, das Abtriebsglied 14 nimmt seine distale, durch den Ausschüttanschlag 2b vorgegebene Endposition und das Antriebsglied 20 nimmt die Ausschüttposition ein. Die Anzeigen 6 und 7 zeigen jeweils die Minimaldosis an.

In diesem Ausgangszustand stellt der Benutzer die gewünschte Produktdosis ein, indem er das Dosierglied 25 mittels Dosierdrehbewegung in die gewünschte Auswahlposition dreht. In der Auswahlposition liegen sich der zugeordnete Dosieranschlag 26 und der Gegenanschlag 17 des Abtriebsglieds 14 axial gegenüber. Der lichte Abstand zwischen den beiden Anschlägen 17 und 26 entspricht der axialen Länge des Dosierhubs.

Nach der Einstellung der Dosis greift der Benutzer das Antriebsglied 20 am Betätigungsteil 23 und zieht es gegen die Vortriebsrichtung V aus dem Gehäuseteil 2 bzw. dem hülsenförmigen Dosierglied 25 bis in den Halteeingriff der Eingriffselemente 24 mit den Eingriffsgegenelementen 27 heraus. Bei diesem Hub nimmt das Antriebsglied 20 im Kupplungseingriff des Kupplungselements 18 und der Kupplungsgegenelemente 22 das Abtriebsglied 14 mit, bis dieses den Dosierhub ausgeführt hat, d. h. der Gegenanschlag 17 in Anschlagkontakt mit dem in der Auswahlposition axial gegenüberliegenden Dosieranschlag 26 gelangt. Wenn dieser Anschlagkontakt hergestellt, der Dosierhub somit beendet ist, und der Benutzer weiterhin am Antriebsglied 20 zieht, löst sich automatisch der Kupplungseingriff 18, 22 und das Antriebsglied 20 legt die restliche Wegstrecke seines axialen Hubs ohne das Abtriebsglied 14 zurück, bis es in die in Figur 2 dargestellte Auslöseposition gelangt. Bei dieser Hubbewegung wird die Antriebsfeder 30 gespannt. Der Halteeingriff 24, 27 ist ausreichend stark bemessen, damit er sich nicht selbsttätig aufgrund der Federkraft der Antriebsfeder 30 lösen kann.

Im gespannten Zustand, d. h. mit dem in der Auslöseposition befindlichen Antriebsglied 20, setzt der Benutzer die Vorrichtung an einer gewünschten Injektionsstelle an, sticht eine am distalen Ende der Vorrichtung angeordnete Injektionsnadel durch die Haut in das subkutane Gewebe und löst dann die Vorrichtung aus. Für die Auslösung drückt der Benutzer durch die als Durchbrüche geformten Eingriffsgegenelemente 27 gegen die Eingriffselemente 24 und drückt dadurch die Eingriffselemente 24 aus dem Halteeingriff. Sobald der Halteeingriff gelöst ist, drückt die Antriebsfeder 30 das Antriebsglied 20 in die Vortriebsrichtung V. Das Antriebsglied 20 drückt aufgrund der Federkraft im Bereich des Flansches 16 gegen das Abtriebsglied 14, so dass das Antriebsglied 20 mit dem Abtriebsglied 14 in die Vortriebsrichtung V bewegt wird. Aufgrund des Zahneingriffs der Mitnehmer 15 werden die Kolbenstange 11 und in der Folge der Kolben 10 im Behältnis 3 in die Vortriebsrichtung V vorgetrieben. Der Hub in die Vortriebsrichtung V setzt sich aus zwei Phasen zusammen. In einer ersten Phase bewegt sich nur das Antriebsglied 20 unter der Federkraft in die Vortriebsrichtung V. Sobald das Antriebsglied 20 in Anschlag gegen das Abtriebsglied 14 gelangt, bewegen sich das Antriebsglied 20 und das Abtriebsglied 14 gemeinsam weiter, bis der Ausschüttanschlag 2b erreicht und dadurch der Hub des Antriebsglieds 20 und der Ausschütthub des Abtriebsglieds 14 beendet ist.

Für den Ausschütthub drückt das Antriebsglied 20 in seinem distalen Bereich, im Ausführungsbeispiel mit seinem distalen Ende, gebildet vom Flansch 16, gegen das Abtriebsglied 14, ebenfalls im Bereich des distalen Endes des Abtriebsglieds 14. Der Mitnahmeeingriff kann aber stattdessen auch an einer anderen Stelle gebildet sein, beispielsweise am proximalen Ende des Abtriebsglieds 14. So können insbesondere eine in die Vortriebsrichtung V weisende innere Anschlagfläche des Betätigungsteils 23, beispielsweise dessen Boden, und eine gegen die Vortriebsrichtung V weisende Endfläche des Endteils 19 in den Mitnahmeeingriff gelangen, beispielsweise die Stirnfläche des Endteils 19. Es müsste nur der axiale Abstand der alternativen Anschlagflächen angepasst werden. Der Mitnahmeeingriff muss nicht durch einen Anschlagkontakt gebildet werden, ein nur auf Druck in die Vortriebsrichtung V belastbarer Anschlagkontakt wird aber nicht nur für das Ausführungsbeispiel, sondern generell bevorzugt.

Bei dem Dosierhub und dem Ausschütthub bewegt sich das vom Abtriebsglied 14 gebildete Eingriffselement 17 zuerst gegen und dann in die Vortriebsrichtung V. Im Führungseingriff 17, 41 versetzt die axiale Hubbewegung des Abtriebsglieds 14 das erste Restmengenglied 40 in eine Drehhubbewegung um die Längsachse L, beim Dosierhub in die eine und beim Ausschütthub in die entgegengesetzte Drehrichtung. Die Restmengenanzeige 7, 42 wird somit bei jedem Ausschütthub wieder zurückgesetzt.

Die eingestellte Dosis wird von der Dosisanzeige 6 angezeigt. Die ausschüttbare Dosis wird von der Restmengenanzeige 7, 42 angezeigt. Solange die ausschüttbare Dosis wenigstens genauso groß wie die eingestellte Dosis ist, zeigen die Anzeigen 6 und 7, 42 die gleichen Dosismengen an. Dies ist beispielsweise in dem in den Figuren 1 und 3 dargestellten Auswahlzustand mit gefülltem Behältnis 3 der Fall.

Unterschreitet die im Behältnis 3 befindliche Produktmenge die eingestellte Dosis, tritt zwischen den Anzeigen 6 und 7, 42 eine Diskrepanz auf. Dieser Zustand ist in den Figuren 2 und 4 dargestellt. Die eingestellte Dosis wird wieder mittels der Anzeige 6 angezeigt. Unmittelbar nach Einstellung der letzten Produktdosis mittels des Doserglieds 25, d. h. noch bevor das Antriebsglied 20 in die Auslöseposition bewegt wurde, zeigt die Restmengenanzeige 7, 42 die Minimaldosis an. Wird das Antriebsglied 20 nun in die Auslöseposition bewegt, die Vorrichtung also aufgezogen, nimmt das Antriebsglied 20 zunächst das Abtriebsglied 14 eine gewisse Wegstrecke mit, die der noch im Behältnis 3 verbliebenen Restmenge des Produkts entspricht. Sobald das Abtriebsglied 14 mit seinem Restmengen-Gegenanschlag 14a in Anschlag gegen den Restmengen-Anschlag 13 der Kolbenstange 11 gelangt, wobei die axiale Anschlagposition des Abtriebsglieds 14 in diesem Fall mit der noch verbliebenen Restmenge des Produkts korrespondiert, löst sich der Kupplungseingriff des Kupplungselements 18 und der Kupplungsgegenelemente 22, und das Antriebsglied 20 bewegt sich bei weiterem Aufbringen axialer Zugkraft alleine, ohne das Abtriebsglied 14 weiter bis in seine Auslöseposition. Die Anzeigen 6 und 7, 42 zeigen nun unterschiedliche Produktmengen an, die Anzeige 6 die eingestellte Produktdosis und die Anzeige 7, 42 die noch verfügbare Restmenge.

Zusätzlich wird die Restmenge durch die axiale Position des Kolbens 10 relativ zur Dosisskala 5 angezeigt. Die von der zweiten Restmengenanzeige 4, 5, 10 angezeigte Restmenge entspricht bei diesem Restfüllstand des Behältnisses 3 der durch die Anzeige 7, 42 angezeigten Restmenge. Solange die im Behältnis 3 insgesamt noch verfügbare Restmenge jedoch die mit dem nächsten Ausschütthub ausschüttbare Restmenge übersteigt, differieren die beiden Restmengenanzeigen voneinander, da die erste Restmengenanzeige 7, 42 die durch den maximalen Ausschütthub ausschüttbare Produktmenge und die zweite Restmengenanzeige 4, 5, 10 die absolut noch verfügbare Restmenge anzeigt.

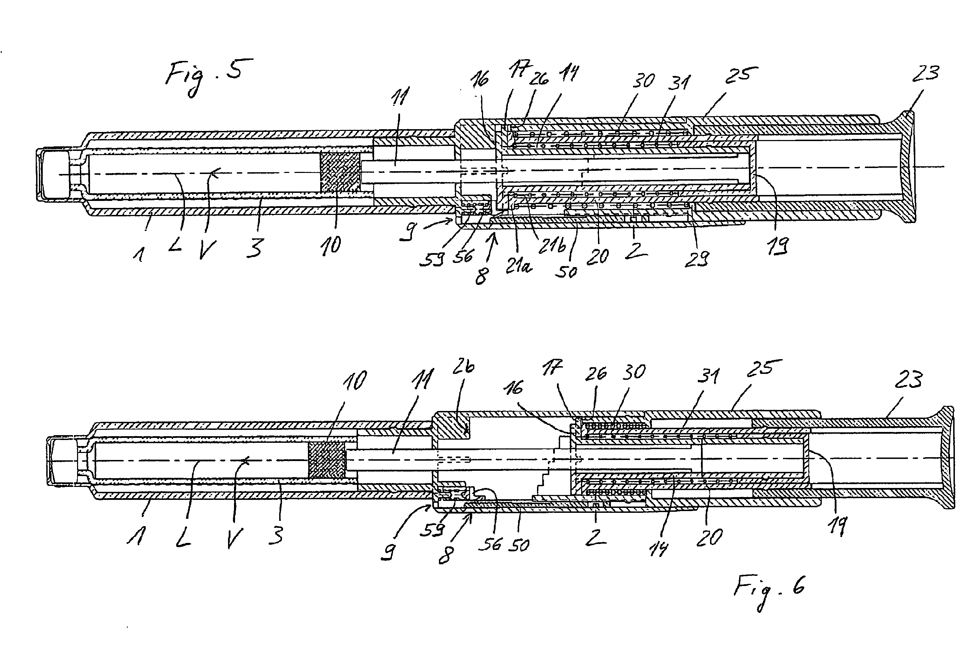

Die Figuren 5 und 6 zeigen eine Vorrichtung eines zweiten Ausführungsbeispiels, die Figur 5 im Auswahlzustand und die Figur 6 im Auslösezustand. Die Vorrichtung des zweiten Ausführungsbeispiels unterscheidet sich von der des ersten Ausführungsbeispiels durch die flexible Kopplung von Abtriebsglied 14 und Antriebsglied 20. Von der Kopplung abgesehen entspricht sie jedoch in Bezug auf die Vorgänge des Dosierens und Förderns der Vorrichtung des ersten Ausführungsbeispiels. So ist beispielsweise die Kolbenstange 11 wieder als Zahnstange gebildet und der Mitnahmeeingriff des Abtriebsglieds 14 und der Kolbenstange 11 dementsprechend ein Zahneingriff. In den Figuren 5 und 6 sind die beiden Zahnreihen 12 des ersten Ausführungsbeispiels nicht zu erkennen, da die Schnittebenen der Figuren 5 und 6 zu denen der Figuren 1 und 2 unter einem rechten Winkel weisen. Die Vorrichtung weist einen Ausschüttungszähler auf, der im ersten Ausführungsbeispiel ebenfalls verwirklicht sein kann. Nachfolgend werden nur die Unterschiede zum ersten Ausführungsbeispiel sowie zum ersten Ausführungsbeispiel noch nicht beschriebene Merkmale, wie beispielsweise der Ausschüttungszähler, beschrieben. Die der Funktion und weitgehend auch der Geometrie nach gleichen Komponenten sind mit den gleichen Bezugszeichen wie im ersten Ausführungsbeispiel versehen.

Die flexible Kopplung des zweiten Ausführungsbeispiels kommt ohne Kupplungseingriff aus. Mit der flexiblen Kopplung des zweiten Ausführungsbeispiels wird die gleiche Wirkung wie im ersten Ausführungsbeispiel erzielt, allerdings mit einer Ausgleichsfeder 31 anstelle des Kupplungseingriffs 18, 22 des ersten Ausführungsbeispiels. Die Ausgleichsfeder 31 wirkt in die Vortriebsrichtung V auf das Antriebsglied 20 und gegen die Vortriebsrichtung V auf das Abtriebsglied 14. Sie ist beispielhaft, wie bevorzugt, in die Vortriebsrichtung V direkt an dem Antriebsglied 20 und gegen die Vortriebsrichtung V direkt an dem Abtriebsglied 14 abgestützt. Alternativ könnte sie aber auch erst über ein oder mehrere Zwischenglieder auf das Antriebsglied 20 oder das Abtriebsglied 14 wirken. Eine direkte Einwirkung durch unmittelbare Abstützung ist jedoch konstruktiv einfacher und erhöht auch die Zuverlässigkeit.

Das Abtriebsglied 14 weist für die Abstützung am äußeren Umfang eine Schulter auf, die im Ausführungsbeispiel vom Endteil 19 gebildet wird, alternativ aber beispielsweise auch am Hülsenabschnitt des Abtriebsglieds 14 als außen umlaufender Flansch geformt sein könnte.

Das Antriebsglied 20 weist wie im ersten Ausführungsbeispiel den Anschlag 21 auf, der sich im zweiten Ausführungsbeispiel allerdings in zwei Schultern 21a und 21b unterteilt. Die Schulter 21a, an der wie im ersten Ausführungsbeispiel die Antriebsfeder 30 abgestützt ist, ragt vom Hülsenabschnitt des Antriebsglieds 20 nach radial außen und die Schulter 21b ragt vom gleichen Hülsenabschnitt auf der gleichen axialen Höhe nach radial innen und bildet innerhalb des Hülsenabschnitts die axiale Abstützung für die Ausgleichsfeder 31. Auf diese Weise entsteht eine platzsparende konzentrisch geschachtelte Anordnung der Federn 30 und 31, wobei die Antriebsfeder 30 in einem äußeren Ringspalt zwischen dem Antriebsglied 20 und dem Dosierglied 25 und die Ausgleichsfeder 31 in einem inneren Ringspalt zwischen dem Abtriebsglied 14 und dem Antriebsglied 20 angeordnet ist. Der genannte Hülsenabschnitt des Antriebsglieds 20 endet in Ausbildung der beiden Schultern 21a und 21b im Längsschnitt gesehen in Form eines "T".

Die Funktionsweise der flexiblen Kopplung ist anhand der Figuren 5 und 6 ersichtlich. Nach der Einstellung der Dosis im Ausgangszustand der Figur 5 wird das Antriebsglied 20 in die Auslöseposition bewegt, die in Figur 6 gezeigt ist. Bei diesem Hub übt das Antriebsglied 20 auf die Ausgleichsfeder 31 eine axiale Druckkraft aus. Die Kraft wird über die Ausgleichsfeder 31 auf das Abtriebsglied 14 übertragen, das somit durch die Federkraft ebenfalls gegen die Vortriebsrichtung V bewegt wird, bis wie im ersten Ausführungsbeispiel der Gegenanschlag 17 gegen den in der Auswahlposition befindlichen Dosieranschlag 26 des Dosierglieds 25 gelangt und der Dosierhub des Abtriebsglieds 14 durch diesen Anschlagkontakt beendet wird. Die Ausgleichsfeder 31 ist ausreichend stark bemessen, um durch vollständigen Hub des Antriebsglieds 20 den Dosierhub des Abtriebsglieds 14 vollständig zu bewirken. Sie weist ferner einen ausreichend langen axialen Federweg auf, um die unterschiedlichen axialen Weglängen des Abtriebsglieds 14 und des Antriebsglieds 20 ausgleichen zu können, nämlich den variablen Dosierhub des Abtriebsglieds 14 und den stets gleich langen Ausziehhub des Antriebsglieds 20 in die Auslöseposition. Die Funktionsweise beim Ausschüttvorgang ist die gleiche wie im ersten Ausführungsbeispiel, d. h. das Antriebsglied 20 drückt über den Anschlag 21a, 21b auf den Flansch 16 des Abtriebsglieds 14 und dadurch dieses in die Vortriebsrichtung V bis gegen den Ausschüttanschlag 2b.

Die Ausgleichsfeder 31 kann auch im ersten Ausführungsbeispiel vorgesehen sein. Sie kann sich dort in gleicher Weise am Abtriebsglied 14, insbesondere an der vom Endteil 19 gebildeten Schulter abstützen. Das Antriebsglied 20 des ersten Ausführungsbeispiels weist auch, wie in den Figuren 1 und 2 erkennbar ist, eine Schulter distal von dem Kupplungsgegenelement 22 auf, an der solch eine Ausgleichsfeder 31 in die Vortriebsrichtung V abgestützt sein kann. Durch Anordnung einer Ausgleichsfeder 31 auch im ersten Ausführungsbeispiel wird die Sicherheit erhöht, dass das Antriebsglied 14 beim Hub des Antriebsglieds 20 den vollständigen Dosierhub ausführt, erforderlich ist eine Ausgleichsfeder 31 im ersten Ausführungsbeispiel allerdings nicht.

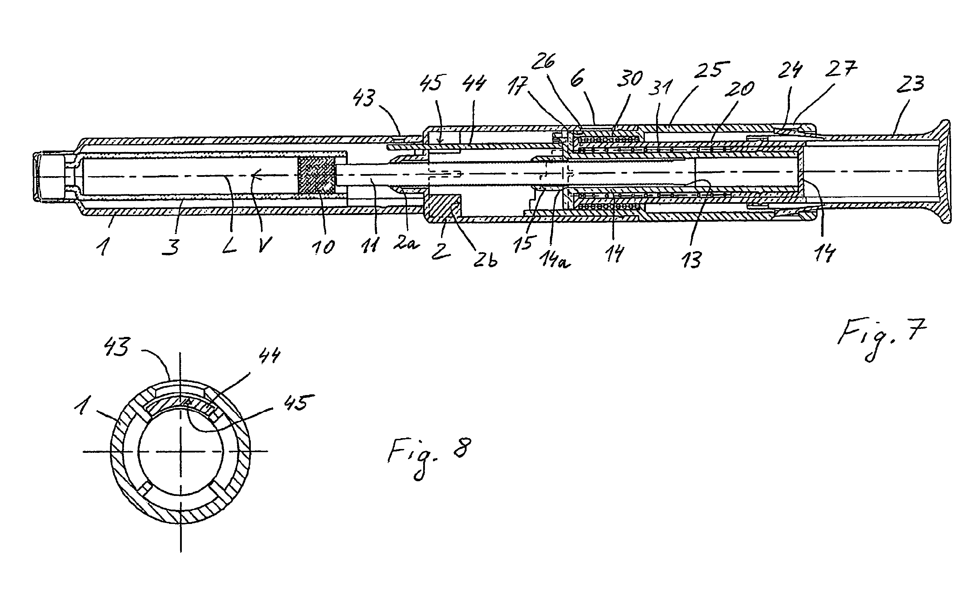

Figur 7 zeigt die Vorrichtung des zweiten Ausführungsbeispiels im Auslösezustand in einem zum Schnitt der Figuren 5 und 6 senkrechten Längsschnitt. Die Vorrichtung weist eine mit dem Abtriebsglied 14 gekoppelte, ebenfalls rücksetzbare Restmengenanzeige nach einem zweiten Ausführungsbeispiel auf. Die rücksetzbare Restmengenanzeige des zweiten Ausführungsbeispiels umfasst ein bewegliches erstes Restmengenglied 44, das im Unterschied zum ersten Ausführungsbeispiel jedoch nicht über ein Getriebe, sondern fest mit dem Abtriebsglied 14 gekoppelt ist. Das Restmengenglied 44 ist relativ zu dem Abtriebsglied 14 axial nicht beweglich, im Ausführungsbeispiel ist es fest mit dem Abtriebsglied 14 verbunden, also relativ zu diesem unbeweglich. Das Restmengenglied 44 ragt von dem Abtriebsglied 14 in Vortriebsrichtung V vor und in die vom Gehäuseteil 1 für das Behältnis 3 gebildete Aufnahme hinein. Auch im zweiten Ausführungsbeispiel bildet das Gehäuse 1, 2 ein zweites Restmengenglied der Restmengenanzeige, indem das Gehäuseteil 1 mit einem Sichtfenster 43 versehen ist, durch das eine Restmengen-Dosisskala 45 des beweglichen ersten Restmengenglieds 44 ablesbar ist. Die Dosisskala 45 ist im zweiten Ausführungsbeispiel aufgrund der axialen Hubbewegungen des Restmengenglieds 44 axial erstreckt, d.h. die Dosismarken der Dosisskala 45 sind axial hintereinander angeordnet. Die Dosismarken sind wie im ersten Ausführungsbeispiel Dosiswerte in Form von Dosiseinheiten repräsentierenden Zahlenwerten.

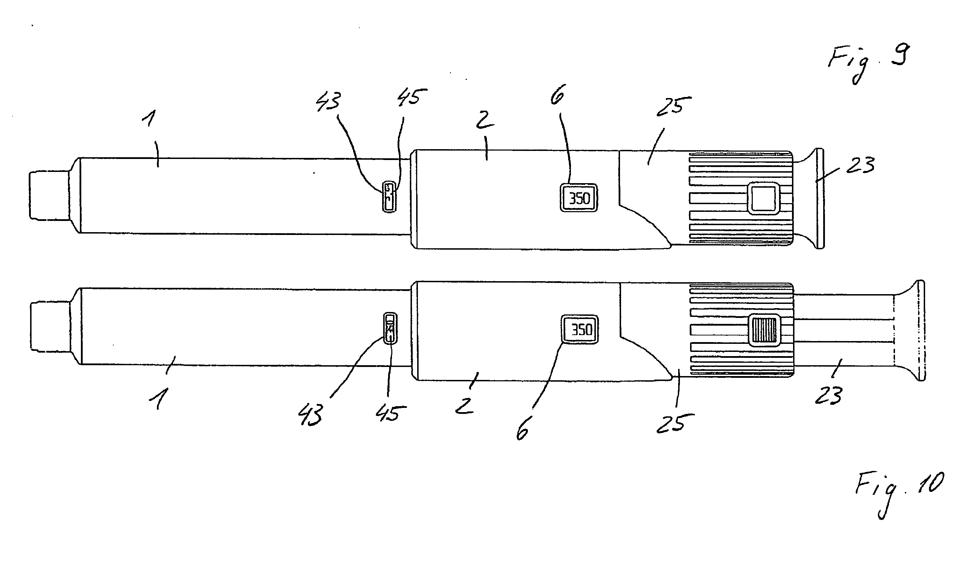

Die Figuren 9 und 10 zeigen die Vorrichtung des zweiten Ausführungsbeispiels in jeweils einer Seitenansicht auf die Dosisanzeige 6 und die Restmengenanzeige 43, 45. In Figur 9 befindet sich die Vorrichtung in einem Auswahlzustand, allerdings wurde mit dem Dosierglied 25 bereits die Produktdosis für die nächste Ausschüttung eingestellt. Sie kann im Sichtfenster 6 abgelesen werden. Solange das Antriebsglied 20 noch die Auswahlposition einnimmt, wie in Figur 9, erscheint im Sichtfenster 43 die Minimaldosis. Nach dem Aufziehen der Vorrichtung erscheint im Auslösezustand, den die Figuren 7 und 10 zeigen, bei ausreichendem Füllstand des Behältnisses 3 der Dosiswert der eingestellten Produktdosis auch im Sichtfenster 43 der Restmengenanzeige 43, 45. Unterschreitet die in Behältnis 3 noch befindliche Produktmenge jedoch die eingestellte Dosis, differieren die beiden angezeigten Dosiswerte entsprechend, indem durch das Sichtfenster 6 die eingestellte Produktdosis und durch das Sichtfenster 43 die nur noch verfügbare, kleinere Restmenge angezeigt wird.

Figur 8 zeigt die Vorrichtung des zweiten Ausführungsbeispiels in einem Querschnitt auf der axialen Höhe des Sichtfensters 43. Die Kolbenstange 11 und die Rückhalteeinrichtung 2a sind allerdings nicht dargestellt. Das Restmengenglied 44 ist in Vortriebsrichtung V lang gestreckt stabförmig. Es ist im Querschnitt an das hülsenförmige Gehäuseteil 1 und das Behältnis 3 angepasst geformt, so dass es in die Vortriebsrichtung V in einen zwischen dem Behältnis 3 und dem Gehäuseteil 1 verbleibenden Ringspalt ragen kann. Im Ausführungsbeispiel weist es im Querschnitt die Form eines Kreissegments auf. Die Anordnung des beweglichen Restmengenglieds 44 in axialer Verlängerung des Abtriebsglieds 14 und wie im zweiten Ausführungsbeispiel vorzugsweise über einen größeren Teil seiner axialen Länge in der vom Gehäuseteil 1 gebildeten Aufnahme für das Behältnis 3 ist vorteilhaft für schlanke Injektionsgeräte.

### Ausschüttunszähler

Die Vorrichtung des zweiten Ausführungsbeispiels ist ferner mit einem Ausschüttungszähler eines in Bezug auf den Zähler ersten Ausführungsbeispiels ausgestattet, der die Anzahl der durchgeführten Ausschüttungen, genauer gesagt der vom Abtriebsglied 14 durchgeführten Ausschütthübe zählt und zur Anzeige bringt.

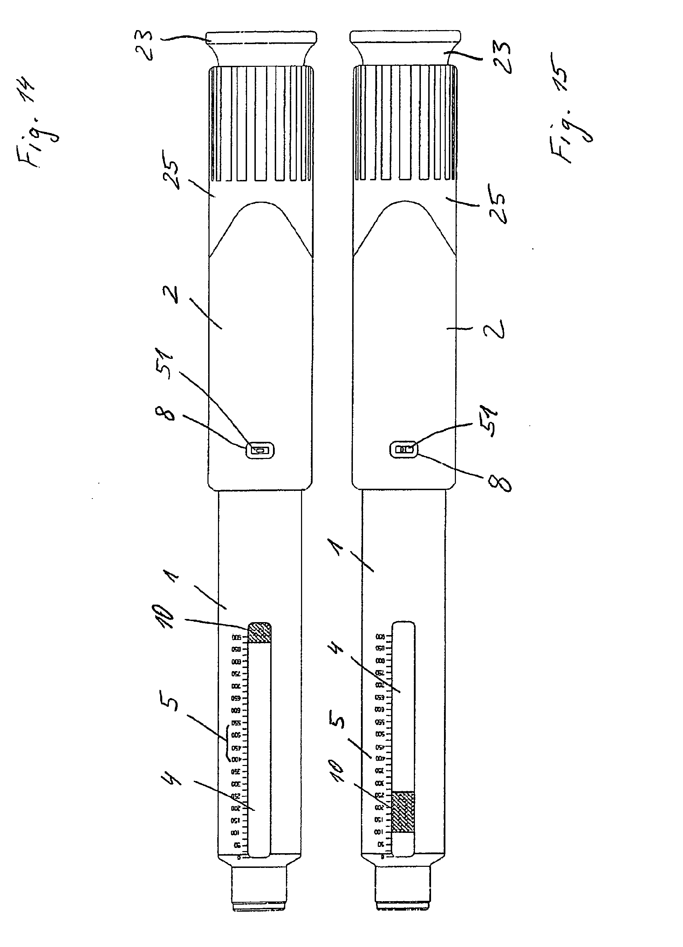

Der Ausschüttungszähler ist auch in den Figuren 14 und 15 erkennbar. Die Figuren 14 und 15 zeigen die Vorrichtung des zweiten Ausführungsbeispiels in der Sicht auf eine von diesem Ausschüttungszähler gebildete Ausschüttungsanzeige 8, 51.

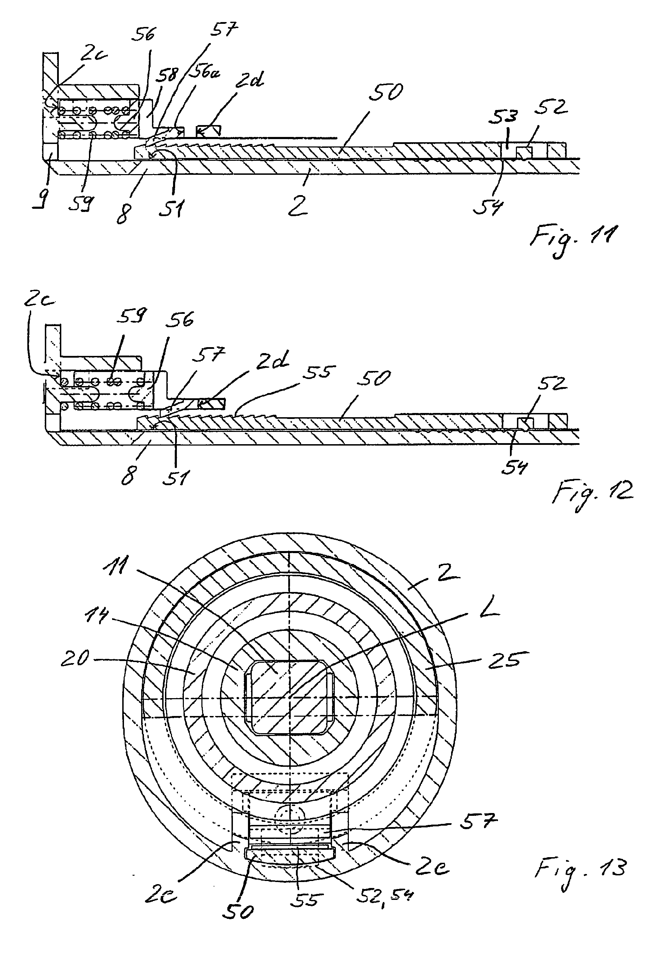

Die Figuren 11 bis 13 zeigen den Ausschüttungszähler des ersten Ausführungsbeispiels, d. h. den Ausschüttungszähler der Vorrichtung nach dem zweiten Ausführungsbeispiel, in jeweils einer vergrößerten Darstellung in einem Längsschnitt. Figur 11 zeigt den Ausschüttungszähler vor einem ersten Dosierhub des Abtriebsglieds 14, und Figur 12 zeigt ihn nach dem ersten Dosierhub, aber noch vor einem ersten Ausschütthub des Abtriebsglieds 14. Figur 13 zeigt den Ausschüttungszähler in einem Querschnitt.

Der Ausschüttungszähler umfasst ein Zählglied 50, das in eine Zählrichtung, im Ausführungsbeispiel die Vortriebsrichtung V, beweglich ist und an einer äußeren Umfangsfläche eine in Zählrichtung V erstreckte Zählskala 51 in fortlaufender Nummerierung beginnend mit der Zahl Null aufweist. Das Zählglied 50 ist so angeordnet, dass die Zählskala 51 durch das Sichtfenster 8 abgelesen werden kann. Das Zählglied 50 ist in die Zählrichtung V zwischen Rastpositionen, d. h. von einer Rastposition in die in Zählrichtung V jeweils nächste Rastposition beweglich und wird durch einen Rasteingriff in der jeweiligen Rastposition gehalten. Für den Rasteingriff weist das Zählglied 50 ein Rastelement 52 auf, das als biegeelastisch nachgiebiger Schnapper gebildet ist. Zur Ausbildung des Rastelements 52 ist das Zählglied 50 mit einer Ausnehmung 53 versehen, in die das Rastelement 52 vorragt. Das Gehäuseteil 2 ist mit Rastgegenelementen 54 versehen, die in Zählrichtung V in einer Reihe hintereinander und voneinander beabstandet angeordnet sind. Die Rastgegenelemente 54 sind an einer Mantelinnenfläche des Gehäuseteils 2 jeweils als eine Rastvertiefung gebildet. Das Rastelement 52 ragt in die der jeweiligen Rastposition zugeordnete Vertiefung 54 hinein. Der Rasteingriff ist mittels einer in die Zählrichtung V wirkenden Kraft lösbar.

Der Ausschüttungszähler umfasst ferner ein Reversierglied 56, das relativ zu dem Gehäuseteil 2 in und gegen die Zählrichtung V hin und her beweglich ist und bei diesen Hubbewegungen vom Gehäuseteil 2 linear geführt wird. Das Reversierglied 56 wird von einem Rückstellglied 58, das vorzugsweise wie im Ausführungsbeispiel von einer mechanischen Feder gebildet wird, gegen die Vortriebs- und Zählrichtung V mit einer Federkraft beaufschlagt. Das Zählglied 50 ist mit dem Reversierglied 56 mittels eines direkten Eingriffs dieser beiden Glieder 50 und 56 gekoppelt. Für den direkten Koppeleingriff weist das Zählglied 50 Eingriffselemente 55 in Form einer in Zählrichtung V erstreckten Zahnreihe und das Reversierglied 56 ein Eingriffsgegenelement 57 in Form eines elastisch in und aus dem Koppeleingriff mit dem Eingriffselement 55 biegbaren Schnappers auf. Die Eingriffselemente 55 sind gepfeilte Zähne mit jeweils einer bezüglich der Zählrichtung V geneigten Vorderflanke und einer im Vergleich dazu steileren Rückflanke. Bei einem Hub des Reversierglieds 56 gegen die Vortriebsrichtung V gleitet das Eingriffsgegenelement 57 über die von den Eingriffselementen 55 gebildete Zahnreihe in den jeweils nächsten Zahneingriff. Bei einem Hub in die Vortriebsrichtung V stemmt sich das Eingriffsgegenelement 57 gegen die steile Rückflanke des jeweiligen Eingriffselements 55 und nimmt das Zählglied 50 daher in die Vortriebs- und Zählrichtung V mit.

Die Eingriffselemente 55 und die Rastgegenelemente 54 sind aufeinander abgestimmt. Die in Vortriebsrichtung V gemessenen Abstände jeweils nächstbenachbarter Eingriffselemente 55 sind so groß wie die Abstände zwischen jeweils zwei nächstbenachbarten Rastgegenelementen 54. Die Haltekraft des Rasteingriffs 52, 54 ist so bemessen, dass sich der Rasteingriff 52, 54 löst, wenn das Reversierglied 56 den Hub in die Vortriebs- und Zählrichtung V ausführt, andererseits aber das Zählglied 50 im jeweiligen Rasteingriff 52, 54 relativ zum Gehäuseteil 2 unbeweglich hält, wenn das Reversierglied 56 den Reversierhub gegen die Vortriebsrichtung V ausführt, d. h. der Rasteingriff 52, 54 hält der bei Ausführung des Reversierhubs vom Eingriffselement 57 auf das Zählglied ausgeübten Kraft stand.

Der reversierende Hub des Reversierglieds 56 wird in und gegen die Vortriebsrichtung V jeweils durch einen Anschlag begrenzt, d. h. der Hub und der gleichlange Reversierhub weisen eine vorgegebene konstante axiale Länge auf. Das Gehäuseteil 2 oder eine damit axial unbeweglich verbundene Komponente bildet einen den reversierenden Hub begrenzenden Anschlag 2d. Bei dem Reversierhub gelangt das Reversierglied 56 mit einer Anschlagfläche 57a gegen den Anschlag 2d. Die Länge des reversierenden Hubs ist geringfügig größer als die Zahnteilung der Reihe von Eingriffselementen 55, beträgt also etwa eine Zahnlänge. Grundsätzlich wäre es auch möglich, dass die Länge des reversierenden Hubs ein ganzzahliges Vielfaches der Zahnlänge plus einer Toleranzzugabe beträgt. Den Hub in die Vortriebsrichtung V begrenzt der Anschlag 2b (Figuren 6 und 7), gegen den das Abtriebsglied 14 in Anschlagkontakt gelangt. Alternativ könnte das Reservierglied 56 in die Vortriebsrichtung V bei 2c gegen den Gehäuseteil 2 in Anschlagkontakt gelangen und damit auch den Ausschütthub des Abtriebsglieds 14 begrenzen. Ein Anschlagkontakt unmittelbar des Abtriebsglieds 14 wird jedoch bevorzugt.

In dem in den Figuren 5 und 11 dargestellten Auswahlzustand drückt das Abtriebsglied 14 in die Vortriebsrichtung V, d. h. in die Zählrichtung, gegen das Reversierglied 56. Im Ausführungsbeispiel drückt das Abtriebsglied 14 mit seinem vorderen Ende, beispielhaft als Flansch 16 gebildet, gegen einen Anschlag 56a des Reversierglieds 56.

Nach Auswahl der Dosis wird das Antriebsglied 20 in die in Figur 6 eingenommene Auslöseposition bewegt. Aufgrund der Kopplung mittels der Ausgleichsfeder 31 wird das Abtriebsglied 14 nachgeführt. Das Reversierglied 56 führt dabei aufgrund der Federkraft des Rückstellglieds 59 den Reversierhub gegen die Vortriebsrichtung V bis in die von dem Anschlag 2d vorgegebene Endposition aus. Das Eingriffsgegenelement 57 rastet dabei in den Koppeleingriff mit der nächsten Zahnlücke des Eingriffselements 35. Diesen Zustand vor Auslösung der Vorrichtung zeigen die Figuren 6 und 12. Wird die Vorrichtung jetzt ausgelöst, nämlich durch Lösen des Eingriffs der Eingriffselemente 24 und Eingriffsgegenelemente 27 (Figur 7), gelangt das Abtriebsglied 14 am Ende seines Ausschütthubs in Anschlagkontakt mit dem Reversierglied 56 und drückt dieses in die Vortriebsrichtung V bis in dessen vordere, durch den Anschlag 2c vorgegebene Endposition. Das Reversierglied 56 nimmt bei diesem Hub aufgrund des Koppeleingriffs 55, 57 das Zählglied 50 in die Vortriebsrichtung V mit. Dieser Zählhub des Zählglieds 50 weist die gleiche Länge wie der Hub des Reversierglieds 56 auf.

Figur 14 zeigt die Vorrichtung mit dem Ausschüttungszähler des ersten Ausführungsbeispiels in einer Seitenansicht auf die mit dem Ausschüttungszähler gebildete Ausschüttungs-Zählanzeige 8, 51. Die Vorrichtung befindet sich im Auswahlzustand vor einer ersten Ausschüttung, d. h. im gleichen Zustand wie in den Figuren 5 und 11. Im Sichtfenster 8 erscheint daher auf der Zählerskala 51 der Zählerstand "0".

Figur 15 zeigt die Vorrichtung nach Ausführung von insgesamt zehn Ausschüttungen. Die Ausschüttungs-Zählanzeige 8, 51 zeigt in ihrem Sichtfenster 8 daher den Zählerstand "10" an.

Die Vorrichtung der Figuren 14 und 15 entspricht der Vorrichtung der Figuren 5 bis 10 mit Ausnahme einer Modifikation hinsichtlich der Anzeige der Restmenge. Die Modifikation besteht darin, dass die Vorrichtung eine zweite Restmengenanzeige 4, 5, 10 aufweist, die der Restmengenanzeige 4, 5, 10 des ersten Ausführungsbeispiels (Figuren 1-4) entspricht. Die Ausschüttungs-Zählanzeige 8, 51 und die Restmengenanzeige 4, 5, 10 sind axial in einer Flucht oder zumindest im Wesentlichen in einer axialen Flucht angeordnet, so dass bequem und eindeutig mit einem Blick gleichzeitig der Zählstand der Ausschüttungs-Zählanzeige 8, 51 und die noch verfügbare Restmenge, hier unmittelbar am Füllstand des Behältnisses 3, abgelesen werden können. Durch Vergleich der angezeigten Restmenge und der angezeigten Anzahl der Ausschüttvorgänge wird eine zusätzliche Möglichkeit der Kontrolle der Korrektheit der Einstellung und Ausschüttung der Dosis und dementsprechend der Therapie geschaffen.

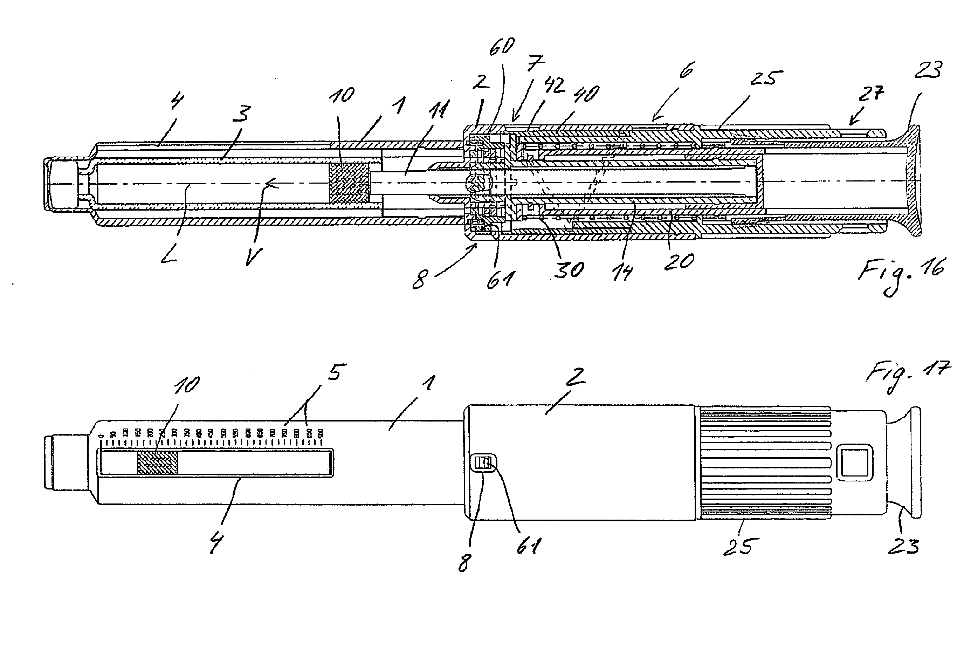

Figur 16 zeigt eine Vorrichtung eines dritten Ausführungsbeispiels mit einem Ausschüttungszähler eines zweiten Ausführungsbeispiels. Die Vorrichtung des dritten Ausführungsbeispiels ist von der Vorrichtung des ersten Ausführungsbeispiels abgeleitet. Sie weist zusätzlich zum ersten Ausführungsbeispiel einen Ausschüttungszähler nach einem zweiten Ausführungsbeispiel auf. Von dieser Ergänzung abgesehen ist sie mit der Vorrichtung des ersten Ausführungsbeispiels identisch.

Der Ausschüttungszähler des zweiten Ausführungsbeispiels umfasst ein Zählglied 60, das in eine Zählrichtung, die quer zur Vortriebsrichtung V weist, bei jedem Ausschütthub des Abtriebsglieds 14 um jeweils einen Zählhub weiterbewegt wird. Das Zählglied 60 ist um die Längsachse L drehbeweglich, der Zählhub ist ein Drehhub. Das Zählglied 60 ist als Zählring geformt. Es weist an einer äußeren Umfangsfläche eine Zählerskala 61 auf, die aus in Zählrichtung, d. h. in Umfangsrichtung, hintereinander angeordneten Zahlen beginnend mit "0" in fortlaufender Nummerierung besteht. In Figur 16 nimmt die Vorrichtung einen Auswahlzustand vor einer ersten Dosisauswahl ein.

Figur 17 zeigt die Vorrichtung der Figur 16 in einer Seitenansicht auf die mit dem Ausschüttungszähler gebildete Ausschüttungs-Zählanzeige 8, 61 des zweiten Ausführungsbeispiels. In Figur 17 ist die Vorrichtung nach zehn Ausschüttvorgängen dargestellt. Entsprechend erscheint im Sichtfenster 8 der Ausschüttungs-Zählanzeige 8, 61 der Zählerstand "10" auf der Zählerskala 61. Die Vorrichtung weist wie die Vorrichtungen der Figuren 14 und 15 eine zweite Restmengenanzeige 4, 5, 10 auf. Die Vorrichtung der Figur 17 ist in Bezug auf die Restmengenanzeige 4, 5, 10 gegenüber der Vorrichtung der Figur 16 modifiziert. Die Modifikation besteht darin, dass die Restmengenanzeige 4, 5, 10 axial nicht wie in Figur 16 in der Verlängerung der ersten Restmengenanzeige 7, 42, sondern in der Verlängerung der Ausschüttungs-Zählanzeige 8, 61 angeordnet ist, wie dies bevorzugt wird. Die Restmengenanzeige 4, 5, 10 entspricht wieder dem ersten Ausführungsbeispiel und ist relativ zu der Ausschüttungs-Zählanzeige 8, 61 wie bei der Vorrichtung der Figuren 14 und 15 angeordnet, so dass die beiden Anzeigen bequem und sicher mit einem Blick abgelesen werden können, d. h. ohne die Vorrichtung zwischen den Ablesevorgängen drehen zu müssen.

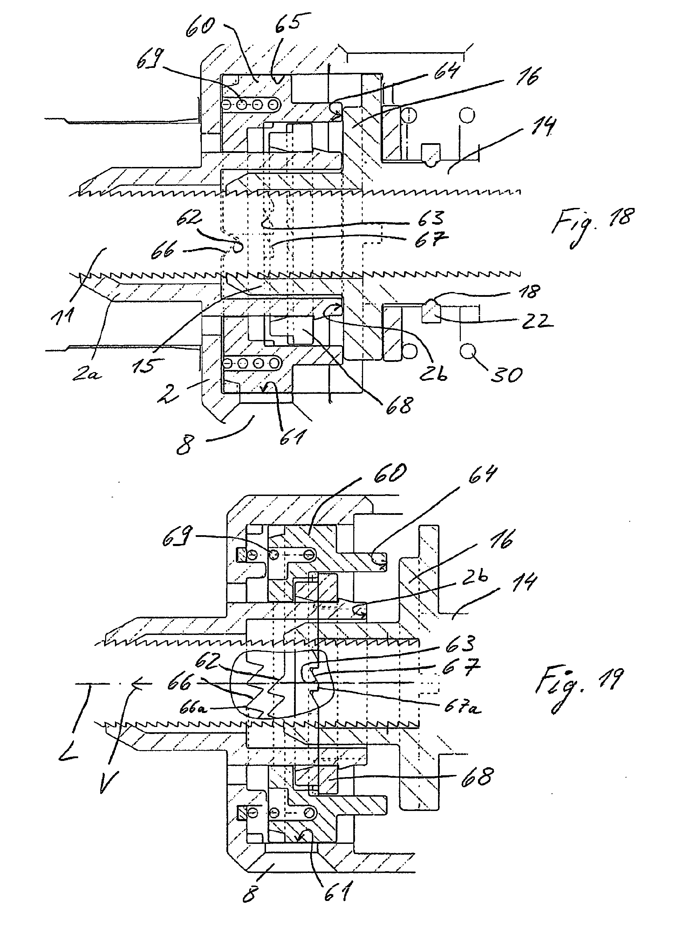

In den Figuren 18 und 19 ist der Ausschüttungszähler der Figur 16 in zwei unterschiedlichen Zuständen vergrößert dargestellt. Der Ausschüttungszähler unterscheidet sich von dem Ausschüttungszähler der Figuren 5 bis 15 nicht nur in der Zählrichtung des Zählglieds 60, sondern auch hinsichtlich des Reversierglieds, das in diesem Ausführungsbeispiel für den Ausschüttungszähler vom Zählglied 60 gebildet wird. Das Zählglied 60 ist dementsprechend nicht nur in die Zählrichtung bewegbar, sondern zusätzlich auch in die Richtung der reversierenden Hubbewegung, die auch im zweiten Ausführungsbeispiel für den Ausschüttungszähler mit der Vortriebsrichtung V übereinstimmt. Das Zählglied 60 führt vergleichbar dem Reversierglied 56 des ersten Ausführungsbeispiels einen Hub in die Vortriebsrichtung V und einen gleichlangen Reversierhub gegen die Vortriebsrichtung V aus und wird bei dieser reversierenden Hubbewegung in die Zählrichtung weiterbewegt. Ein erster Teil jedes Zählhubs wird durch den Reversierhub, also den Hub gegen die Vortriebsrichtung V, und der verbleibende zweite Teil jedes Zählhubs wird durch den Hub in die Vortriebsrichtung V bewirkt. Den Hub in die Vortriebsrichtung V bewirkt das Abtriebsglied 14 bei jedem Ausschütthub, indem es mit seinem vorderen Ende, beispielhaft dem Flansch 16, in einer Endphase des jeweiligen Ausschütthubs in einen Anschlagkontakt gegen einen Anschlag 64 des Zählglieds 60 gelangt und dieses im Anschlagkontakt in die Vortriebsrichtung V drückt. Dem Abtriebsglied 14 ist entgegenwirkend ein Rückstellglied 69 angeordnet, das auch beim Ausschüttungszähler des zweiten Ausführungsbeispiels vorzugsweise als mechanische Feder, beispielhaft wieder als Druckfeder, gebildet ist. Das Rückstellglied 69 beaufschlagt das Zählglied 60 mit einer gegen die Vortriebsrichtung V wirkenden Federkraft.

Figur 18 zeigt den Ausschüttungszähler im gleichen Zustand wie Figur 16. Das Zählglied 60 nimmt in Bezug auf die Vortriebsrichtung V eine vordere Endposition ein. Wird das Antriebsglied 20 nach Auswahl der Dosis gegen die Vortriebsrichtung V in die Auslöseposition bewegt und dabei das Abtriebsglied 14 aufgrund der Kopplung 18, 22 mitgenommen, führt das Zählglied 60 unter der Federkraft des Rückstellglieds 69 automatisch den Reversierhub bis in eine durch Anschlagkontakt vorgegebene hintere Endposition ein. Diesen Zustand des Ausschüttungszählers mit dem in der hinteren Endposition befindlichen Zählglied 60 zeigt die Figur 19.

Das Zählglied 60 weist erste Eingriffselemente 62 auf, die bei dem Hub in die Vortriebsrichtung V mit ersten Führungselementen 66 in einen Koppeleingriff gelangen, der die Hubbewegung des Zählglieds 60 in eine Drehhubbewegung in Zählrichtung umwandelt. Ferner weist das Zählglied 60 zweite Eingriffselemente 63 auf, die bei dem Reversierhub mit zweiten Führungselementen 67 in einen zweiten Koppeleingriff gelangen, der die Hubbewegung gegen die Vortriebsrichtung V ebenfalls in einen Drehhub in Zählrichtung umwandelt. Ein kompletter Zählhub, bei dem das Zählglied 60 um einen Zähler in die Zählrichtung weiterbewegt wird, setzt sich aus den beiden Teildrehhüben zusammen. Die Eingriffselemente 62 und die damit zusammenwirkenden Führungselemente 66 weisen zur Vortriebsrichtung V und auch zur Zählrichtung geneigte Eingriffsflanken auf, mit denen sie im Koppeleingriff aneinander abgleiten und dadurch die Umwandlung der in die Vortriebsrichtung V gerichteten Relativbewegung des Zählglieds 60 in den Drehhub in die Zählrichtung umwandeln. Ebenso sind die Eingriffselemente 63 und die damit zusammenwirkenden Führungselemente 67 sowohl bezüglich der Vortriebsrichtung V als auch der Zählrichtung geneigt, um in analoger Weise den Reversierhub des Zählglieds 60 in den anderen Drehhubteil des Zählhubs umzuwandeln.

Im Ausführungsbeispiel werden die Eingriffselemente 62 und 63 und die Führungselemente 66 und 67 jeweils von einer Pfeilverzahnung gebildet. Die Eingriffselemente 62 und 63 sind unmittelbar am Zählglied 60 geformt, beispielhaft in der Art eines Zahnkranzes, d. h. mit in Vortriebsrichtung V vorstehenden Eingriffselementen 62 und gegen die Vortriebsrichtung V vorstehenden Eingriffselementen 63. Die Führungselemente 66 und 67 sind jeweils kongruent geformt, die Führungselemente 66 am Gehäuseteil 2 und die Führungselemente 67 an einem Führungseinsatz 68, der beispielhaft als Führungsring geformt ist. Der Führungseinsatz 68 ist gegen die Vortriebsrichtung V an dem Gehäuseteil 2 abgestützt, beispielhaft formschlüssig durch Anschlagkontakt. Der Funktion nach könnte er mit dem Gehäuseteil 2 in einem Stück geformt sein, die separate Formung hat fertigungstechnische Gründe.

Der reversierende Hub des Zählglieds 60 wird durch Anschläge begrenzt, in Vortriebsrichtung V durch einen Anschlag des Gehäuseteils 2, beispielsweise einem Zahngrund 66a der Führungselemente 66 oder bevorzugt durch den Anschlag 2b für das Abtriebsglied 14, und gegen die Vortriebsrichtung V durch einen Anschlag des Führungseinsatzes 68, beispielsweise einen Zahngrund 67a der Führungselemente 67. Das Zählglied 60 ist in Zählrichtung zwischen Rastpositionen beweglich, die in Zählrichtung der Länge des jeweiligen Teildrehhubs entsprechend voneinander beabstandet sind. In den Rastpositionen ist es in und gegen die Vortriebsrichtung V geführt. Beispielhaft ist der Rasteingriff im Bereich des äußeren Umfangs des Zählglieds 60 bei 65 gebildet.

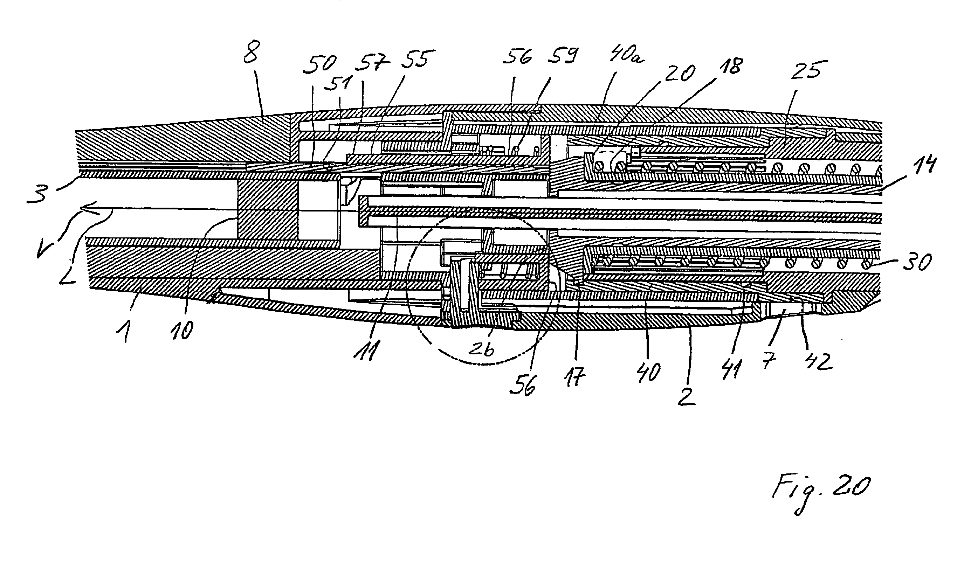

Figur 20 zeigt eine Vorrichtung mit einer Fördereinrichtung und einer Restmengenanzeige, die im Wesentlichen denen des ersten Ausführungsbeispiels (Figuren 1-4) entsprechen. Das Abtriebsglied 14 und das Antriebsglied 20 sind mittels einer Kupplung miteinander gekoppelt, die in gleicher Weise wie im ersten Ausführungsbeispiel mit einem Kupplungselement 18 und Kupplungsgegenelementen 22 gebildet ist. In Figur 20 ist lediglich das Kupplungselement 18 zu erkennen, die Kupplungsgegenelemente 22 sind außerhalb der Längsschnittebene der Figur 20 im Kupplungseingriff mit dem Kupplungselement 18. Ein Unterschied zum ersten Ausführungsbeispiel besteht darin, dass das Antriebsglied 20 mit am Außenumfang des Abtriebsglieds 14 im Gleitkontakt anliegt, wodurch das Gerät schlanker gehalten werden kann. Die Restmengenanzeige 7, 42 unterscheidet sich vom ersten Ausführungsbeispiel lediglich dadurch, dass die Restmengen-Dosisskala 42 im Bereich des hinteren Endes des Restmengenglieds 40 angeordnet ist, während sie sich im ersten Ausführungsbeispiel im vorderen Bereich befindet.

Im Unterschied zum ersten Ausführungsbeispiel erfüllt das Restmengenglied 40 für den Ausschüttungszähler des dritten Ausführungsbeispiels allerdings die Funktion eines Anschlags, der die reversierende Hubbewegung des Reversierglieds 56 begrenzt. Das Restmengenglied 40 selbst ist wie im ersten Ausführungsbeispiel um die Längsachse L nur drehbeweglich, axial relativ zum Gehäuse 1, 2 jedoch unbeweglich. Es bildet mit einer vorderen Stirnfläche einen Anschlag 40a, der den Reversierhub des Reversierglieds 56 gegen die Vortriebsrichtung V begrenzt. Den Hub in die Vortriebsrichtung V beendet der Anschlagkontakt des Abtriebsglieds 14 gegen den Anschlag 2b.

Das Zählglied 50 entspricht im Wesentlichen dem Zählglied 50 des ersten Ausführungsbeispiels (Figuren 11-13), so dass auf die dortigen Ausführungen verwiesen wird. Es ist wie im ersten Ausführungsbeispiel lang gestreckt stabförmig. Es weist über seine gesamte Länge oder zumindest über einen größeren Teil seiner axialen Länge im Querschnitt die Form eines Bogensegments, vorzugsweise Kreisbogensegments auf, wie im ersten Ausführungsbeispiel. Es ragt auch wieder in einen Zwischenraum, der zwischen dem Gehäuseteil 1 und dem Behältnis 3 frei bleibt. Im dritten Ausführungsbeispiel bleibt solch ein Raum allerdings nicht umlaufend um das Behältnis 3 frei, vielmehr ist das Gehäuseteil 1 im Bereich der Aufnahme zur Schaffung des Spalts für das Zählglied 50 im Querschnitt oval geformt, beispielsweise ellipsenförmig oder birnenförmig. Ein Sichtfenster auf die Zählerskala 51 des Zählglieds 50 ist im Gehäuseteil 1 bei 8 gebildet. Es kann sich um einen Durchbruch ohne oder mit durchsichtigem Verschließelement handeln. Alternativ kann das Sichtfenster mittels 2K-Spritzgusstechnik geschaffen werden, was auch für die anderen Beispiele gilt.

Das Reversierglied 56 ist ebenfalls vom ersten Ausführungsbeispiel für den Ausschüttungszähler abgeleitet, im Unterschied dazu umgibt es die Kolbenstange 11. Es wird reversierend hubbeweglich vom Gehäuse 1, 2 linear geführt. Die Linearführung des Gehäuses 1, 2 ragt zentral in das Reversierglied 56. Das Rückstellglied 59 wird wieder von einer mechanischen Druckfeder gebildet, die im dritten Ausführungsbeispiel des Ausschüttungszählers ebenfalls die Kolbenstange 11 umgibt. Das Rückstellglied 59 ist dementsprechend deutlich größer als im ersten Ausführungsbeispiel. Die Größe des Reversierglieds 56 und des Rückstellglieds 59 erleichtern im Vergleich mit dem ersten Ausführungsbeispiel des Ausschüttungszählers die Fertigung und die Montage, ferner die Auslegung des Rückstellglieds 59 in Bezug auf seine Federcharakteristik und die Auslegung des Reversierglieds 56 im Hinblick auf die elastische Nachgiebigkeit seines Eingriffsgegenelements 57 und seine axiale Führung.

### Bezugszeichen:

- 1: Gehäuseteil, Restmengenglied
- 2: Gehäuseteil, Restmengenglied
- 2a: Rückzugssicherung
- 2b: Ausschüttanschlag
- 2c: Anschlag (optional)
- 2d: Anschlag
- 2e: Führung, Einfassung
- 3: Behältnis
- 4: Restmengenanzeige, Sichtfenster
- 5: Dosisskala
- 6: Dosisanzeige, Sichtfenster
- 7: Restmengenanzeige, Sichtfenster
- 8: Ausschüttungsanzeige, Sichtfenster
- 9: Durchgang
- 10: Kolben
- 11: Kolbenstange
- 12: Zahnreihe
- 13: Restmengen-Anschlag
- 14: Abtriebsglied, erstes Förderglied
- 14a: Restmengen-Gegenanschlag
- 15: Mitnehmer
- 16: Flansch
- 17: Dosier-Gegenanschlag, Eingriffselement
- 18: Kupplungselement
- 19: Endteil
- 20: Antriebsglied, zweites Förderglied
- 21: Anschlag, Schulter
- 21a: Schulter
- 21b: Schulter
- 22: Kupplungsgegenelement
- 23: Betätigungsteil
- 24: Eingriffselement
- 25: Dosierglied
- 26: Dosieranschlag
- 27: Eingriffsgegenelement
- 28: Profilierung
- 29: Schulter
- 30: Antriebsfeder
- 31: Ausgleichsfeder
- 32-39 - 40: Restmengenglied
- 40a: Anschlag
- 41: Führungskurve
- 42: Restmengen-Dosisskala
- 43: Sichtfenster
- 44: Restmengenglied
- 45: Restmengen-Dosisskala
- 46-49 - 50: Zählglied
- 51: Zählerskala
- 52: Rastelement
- 53: Ausnehmung
- 54: Rastgegenelement
- 55: Eingriffselemente
- 56: Reversierglied
- 56a: Anschlag
- 57: Eingriffsgegenelement
- 57a: Anschlag
- 58: Ausnehmung
- 59: Rückstellglied
- 60: Zählglied
- 61: Zählerskala
- 62: Eingriffselement
- 63: Eingriffselement
- 64: Gegenanschlag
- 65: Rastelement
- 66: Führungselement
- 66a: Anschlag (optional)
- 67: Führungselement
- 67a: Anschlag
- 68: Führungseinsatz
- 69: Rückstellglied

- L: Längsachse
- V: Vortriebsrichtung

## Patentansprüche

1. Verabreichungsvorrichtung mit Restmengenanzeige, die Vorrichtung umfassend:
a) ein Gehäuse (1, 2) mit einer Aufnahme für ein zu verabreichendes Produkt,
b) eine Kolbenstange (11), die zur Ausschüttung einer Produktdosis in eine Vortriebsrichtung (V) bewegbar ist,
c) ein Dosierglied (25), das zur Einstellung der Produktdosis, relativ zu dem Gehäuse (1, 2) in unterschiedliche Auswahlpositionen verstellbar ist,
d) ein Abtriebsglied (14), das gegen die Vortriebsrichtung (V) einen Dosierhub bis in eine mittels des Dosierglieds (25) vorgegebene Dosisposition und aus der Dosisposition einen Ausschütthub in die Vortriebsrichtung (V) ausführen kann,
e) wobei das Abtriebsglied (14) mit der Kolbenstange (11) so gekoppelt ist, dass der Ausschütthub die Bewegung der Kolbenstange (11) in die Vortriebsrichtung (V) bewirkt,
f) ein erstes Restmengenglied (40; 44), das mit dem Abtriebsglied (14) so gekoppelt ist, dass der Dosierhub eine Bewegung des ersten Restmengenglieds (40; 44) in eine erste Richtung und der Ausschütthub eine Bewegung in die Gegenrichtung bewirkt,
g) und ein zweites Restmengenglied (2; 1), relativ zu dem das erste Restmengenglied (40; 44) die Bewegungen ausführt,
h) wobei eines (40; 44) der Restmengenglieder eine Dosisskala (42; 45) aufweist und aus der Position, die das andere (2; 1) der Restmengenglieder relativ zu der Dosisskala (42; 45) einnimmt, die noch ausschüttbare Restmenge des Produkts ablesbar ist,
**dadurch gekennzeichnet, dass**
i) die Verabreichungsvorrichtung ferner ein das erste Restmengenglied (40) und das Abtriebsglied (14) koppelndes Kurvengetriebe (14, 17, 40, 41) umfasst, das ein Führungsglied mit eine Führungskurve (41) und ein Eingriffsglied mit einem mit der Führungskurve (41) in einem Führungseingriff (17, 40) befindlichen Eingriffselement (17) aufweist, wobei das Kurvengetriebe (14, 17, 40, 41) den Dosierhub und den Ausschütthub des Abtriebsglieds (14) in die Bewegungen des ersten Restmengenglieds (40) umwandelt, zum Beispiel bereits nur dieser Führungseingriff (17, 41) die Hübe in die Bewegungen des ersten Restmengenglieds (40) umwandelt.

2. Verabreichungsvorrichtung nach dem vorhergehenden Anspruch, wobei das Getriebe (14, 17, 40, 41) den Dosierhub und den Ausschütthub des Abtriebsglieds (14) in Bezug auf die Länge übersetzt.

3. Verabreichungsvorrichtung nach dem vorhergehenden Anspruch und wenigstens einem der folgenden Merkmale:
- das Führungsglied und das Eingriffsglied bilden im Führungseingriff (17, 41) ein Schraubgelenk, das bezüglich seiner Drehachse (L) eine Schraubensteigung aufweist, die so groß ist, dass eine im Führungseingriff (17, 41) wirkende axiale Kraft eine Relativdrehung zwischen dem Führungsglied und dem Eingriffsglied bewirkt;
- das erste Restmengenglied (40) erstreckt sich um das Abtriebsglied (14) und ist in einem dem Abtriebsglied (14) zugewandten inneren Umfangsbereich in dem Führungseingriff (17,41), zum Beispiel mit dem Abtriebsglied (14);
- das erste Restmengenglied (40) erstreckt sich um das Abtriebsglied (14) und das Abtriebsglied (14) ist in einem dem ersten Restmengenglied (40) zugewandten äußeren Umfangsbereich in dem Führungseingriff (17, 41), zum Beispiel mit dem ersten Restmengenglied (40);
- das erste Restmengenglied (40) ist um eine Drehachse (L) drehbar und bildet in einem um die Drehachse (L) erstreckten inneren Umfangsbereich entweder das Eingriffsglied oder das Führungsglied des Kurvengetriebes (17, 41).

4. Verabreichungsvorrichtung nach einem der drei vorhergehenden Ansprüche und wenigstens einem der folgenden Merkmale:
- das Abtriebsglied (14) und das erste Restmengenglied (40) bilden das Getriebe (14, 17, 40, 41), vorzugsweise das Kurvengetriebe (14, 17, 40, 41) nach einem der zwei vorhergehenden Ansprüche;
- das erste Restmengenglied (40) ist um eine Drehachse (L) drehbar;
- das erste Restmengenglied (40) ist hülsenförmig.

5. Verabreichungsvorrichtung nach einem der Ansprüche 1 bis 3, wobei das erste Restmengenglied (40) um eine Drehachse (L) drehbar und relativ zu dem Gehäuse (1, 2) axial nicht beweglich ist.

6. Verabreichungsvorrichtung nach einem der vorhergehenden Ansprüche und wenigstens einem der folgenden Merkmale:
- das andere der Restmengenglieder (2; 1) weist ein Sichtfenster (7; 43) auf, durch das die Dosisskala ablesbar ist;
- eines der Restmengenglieder (40, 2) umgibt das andere;
- das erste Restmengenglied (40; 44) weist die Dosisskala auf, zum Beispiel an einer äußeren Umfangsfläche;
- das Gehäuse (1, 2) bildet das zweite Restmengenglied.

7. Verabreichungsvorrichtung nach einem der vorhergehenden Ansprüche ferner umfassend eine Dosisanzeige (6, 25), die stets die eingestellte Produktdosis anzeigt und zum Beispiel eine weitere Dosisskala aufweist.

8. Verabreichungsvorrichtung nach dem vorhergehenden Anspruch und wenigstens einem der folgenden Merkmale:
- die weitere Dosisskala ist durch ein Sichtfenster (7), zum Beispiel ein Sichtfenster (6) des Gehäuses (1, 2) ablesbar;
- eines aus Dosierglied (25) und Gehäuse (1, 2) weist die weitere Dosisskala auf, zum Beispiel das Dosierglied (25), und das andere einen Marker, den zum Beispiel ein Sichtfenster (6) bildet, wobei die eingestellte Produktdosis an der Position ablesbar ist, die der Marker und die Dosisskala relativ zueinander einnehmen;
- die eingestellte Dosis und die noch ausschüttbare Restmenge sind längs einer zu der Vortriebsrichtung (V) parallelen Achse in einem axialen Abstand voneinander an relativ zu dem Gehäuse (1, 2) ortsfesten Positionen auf der gleichen Seite des Gehäuses (1, 2) und somit ohne Verdrehung des Gehäuses (1, 2) gleichzeitig sichtbar.

9. Verabreichungsvorrichtung nach einem der zwei vorhergehenden Ansprüche, **dadurch gekennzeichnet, dass** die eingestellte Dosis durch ein erstes Sichtfenster (6) und die noch ausschüttbare Restmenge durch ein von dem ersten Sichtfenster (6) beabstandetes, zum Beispiel axial beabstandetes zweites Sichtfenster (7; 43) ablesbar ist.

10. Verabreichungsvorrichtung nach einem der vorhergehenden Ansprüche, **dadurch gekennzeichnet, dass**
(i) das Gehäuse (1, 2) im Bereich der Aufnahme durchsichtig ist, zum Beispiel ein Sichtfenster (4) aufweist,
(ii) sich im Bereich der Aufnahme in Vortriebsrichtung (V) eine weitere Dosisskala (5) erstreckt
(iii) und im durchsichtigen Bereich die Position eines das Produkt fördernden Kolbens (10) relativ zu der weiteren Dosisskala (5) erkennbar ist,
(iv) so dass der Kolben (10) für die weitere Dosisskala (5) einen Marker bildet, der an der weiteren Dosisskala (5) die noch ausschüttbare Restmenge anzeigt.

11. Verabreichungsvorrichtung nach einem der vorhergehenden Ansprüche, **dadurch gekennzeichnet, dass** die Kolbenstange (11) einen Restmengen-Anschlag (13) aufweist, gegen den ein Restmengen-Gegenanschlag (14a) des Abtriebsglieds (14) in einen Anschlagkontakt gelangt, der den Dosierhub des Abtriebsglieds (14) begrenzt, wenn die noch ausschüttbare Restmenge kleiner als die eingestellte Produktdosis ist.

12. Verabreichungsvorrichtung nach einem der Ansprüche 1 und 6 bis 11 und ferner wenigstens einem der folgenden Merkmale:
- das erste Restmengenglied (44) ist mit dem Abtriebsglied (14) axial nicht beweglich gekoppelt;
- das erste Restmengenglied (44) ist mit dem Abtriebsglied (14) in einem Stück geformt oder zum Beispiel an dem Abtriebsglied (14) befestigt;
- das erste Restmengenglied (44) ragt von dem Abtriebsglied (14) in die Vortriebsrichtung (V) vor;
- das erste Restmengenglied (44) erstreckt sich in Vortriebsrichtung (V) in die Aufnahme des Gehäuses (1, 2) oder in eine axiale Überlappung mit der Aufnahme;
- das erste Restmengenglied (44) ist stabförmig.

13. Verabreichungsvorrichtung nach einem der vorhergehenden Ansprüche und wenigstens einem der folgenden Merkmale:
- das Abtriebsglied (14) ist mit der Kolbenstange (11) in die Vortriebsrichtung (V) in einem Mitnahmeeingriff gekoppelt und nimmt die Kolbenstange (11) bei dem Ausschütthub mitnimmt;
- die Kolbenstange (11) weist eine axial erstreckte Zahnreihe (12) mit Sägezähnen auf, und das Abtriebsglied (14) ist mit der Kolbenstange (11) durch einen Zahneingriff gekoppelt ist, in dem sich ein Mitnehmer (15) und die Zahnreihe (12) befinden, um die Kolbenstange (11) bei dem Ausschütthub in die Vortriebsrichtung (V) zu bewegen, wobei der Mitnehmer (15) axial nicht beweglich mit dem Abtriebsglied (14) verbunden, zum Beispiel an dem Abtriebsglied (14) geformt ist;
- die Kolbenstange (11) ist an einer Bewegung gegen die Vortriebsrichtung (V) gehindert;
- das Abtriebsglied (14) ist nur in und gegen die Vortriebsrichtung (V) bewegbar.

14. Verabreichungsvorrichtung nach einem der vorhergehenden Ansprüche und wenigstens einem der folgenden Merkmale:
- das Dosierglied (25) bestimmt in den Auswahlpositionen den Dosierhub durch Anschlagkontakt, wobei das Abtriebsglied (14) zum Beispiel unmittelbar in den Anschlagkontakt gelangt;
- das Dosierglied (25) bildet für jede der Auswahlpositionen einen Dosieranschlag (26) und bestimmt den Dosierhub durch Anschlagkontakt;
- das Dosierglied (25) ist relativ zu dem Gehäuse (1, 2) um eine axial erstreckte Drehachse (L) drehbar und weist um die Drehachse (L) verteilt mehrere Dosieranschläge (26) auf axial unterschiedlichen Höhen oder eine kontinuierlich spiralig um die Drehachse (L) erstreckten Dosieranschlag auf, die oder der den Dosierhub durch Anschlagkontakt entsprechend der Auswahlposition des Dosierglieds (25) vorgeben oder vorgibt;
- das Dosierglied (25) ist relativ zu dem Gehäuse (1, 2) axial nicht beweglich;
- das Dosierglied (25) ist ein Hülsenkörper, der das Abtriebsglied (14) oder ein Antriebsglied (20) für das Abtriebsglied (14) umgibt.

15. Verabreichungsvorrichtung nach einem der vorhergehenden Ansprüche, ferner umfassend
(i) ein Antriebsglied (20), das mittels eines axialen Hubs aus einer Ausschüttposition bis in eine Auslöseposition und aus dieser zurück in die Ausschüttposition bewegbar ist,
(ii) wobei das Antriebsglied (20) in wenigstens einer der Auswahlpositionen des Dosierglieds (25) einen längeren Hub als das Abtriebsglied (14) ausführt, zum Beispiel unabhängig von dem Abtriebsglied (14) axial bewegbar ist,
(iii) und eine Kopplung (18, 22; 31), die das Antriebsglied (20) mit dem Abtriebsglied (14) so koppelt, dass auch für die wenigstens eine der Auswahlpositionen der Hub des Antriebsglieds (20) in die Auslöseposition den Dosierhub und der Hub des Antriebsglieds (20) in die Ausschüttposition den Ausschütthub des Abtriebsglieds (14) bewirkt.

## Claims

1. An administration device with a residual amount display, the device including:
a) a housing (1, 2) with a receiving means for a product to be administered,
b) a piston rod (11) which is movable in an advance direction (V) to expel a product dose,
c) a dosing member (25) which is displaceable into different selection positions for adjusting the product dose relative to the housing (1, 2),
d) a driven member (14) which can perform a dosing stroke in opposite relationship to the advance direction (V) into a dose position predetermined by the dosing member (25) and an expulsion stroke in the advance direction (V) from the dose position,
e) wherein the driven member (14) is so coupled to the piston rod (11) that the expulsion stroke causes the movement of the piston rod (11) in the advance direction (V),
f) a first residual amount member (40; 44) so coupled to the driven member (14) that the dosing stroke causes a movement of the first residual amount member (40; 44) in a first direction and the expulsion stroke causes a movement in the opposite direction, and
g) a second residual amount member (2; 1), relative to which the first residual amount member (40; 44) performs the movements,
h) wherein one (40; 44) of the residual amount members has a dose scale (42; 45) and the residual amount of product that can still be expelled can be read off from the position which the other (2; 1) of the residual amount members assumes relative to the dose scale (42; 45),
**characterised in that**
i) the administration device further includes a cam transmission (14, 17, 40, 41) which couples the first residual amount member (40) and the driven member (14) and which has a guide member with a guide cam (41) and an engagement member with an engagement element (17) disposed in a guide engagement (17, 40) with the guide cam (41), wherein the cam transmission (14, 17, 40, 41) converts the dosing stroke and the expulsion stroke of the driven member (14) into the movements of the first residual amount member (40), for example it is already only said guide engagement (17, 41) that converts the strokes into the movements of the first residual amount member (40).

2. An administration device according to the preceding claim wherein the transmission (14, 17, 40, 41) converts the dosing stroke and the expulsion stroke of the driven member (14) in relation to length.

3. An administration device according to the preceding claim and at least one of the following features:
- the guide member and the engagement member form in the guide engagement (17, 41) a screw joint which in relation to its axis of rotation (L) has a screw pitch which is so great that an axial force acting in the guide engagement (17, 41) causes a relative rotation between the guide member and the engagement member;
- the first residual amount member (40) extends around the driven member (14) and is in an inner peripheral region towards the driven member (14) in the guide engagement (17, 41), for example with the driven member (14);
- the first residual amount member (40) extends around the driven member (14) and the driven member (14) is in an outer peripheral region towards the first residual amount member (40) in the guide engagement (17, 41), for example with the first residual amount member (40); and
- the first residual amount member (40) is rotatable about an axis of rotation (L) and in an inner peripheral region extending around the axis of rotation (L) forms either the engagement member or the guide member of the cam transmission (17, 41).

4. An administration device according to one of the three preceding claims and at least one of the following features:
- the driven member (14) and the first residual amount member (40) form the transmission (14, 17, 40, 41), preferably the cam transmission (14, 17, 40, 41) according to one of the two preceding claims;
- the first residual amount member (40) is rotatable about an axis of rotation (L); and
- the first residual amount member (40) is sleeve-shaped.

5. An administration device according to one of claims 1 to 3 wherein the first residual amount member (40) is rotatable about an axis of rotation (L) and is axially immobile relative to the housing (1, 2).

6. An administration device according to one of the preceding claims and at least one of the following features:
- the other of the residual amount members (2; 1) has a sight window (7; 43) through which the dose scale can be read off;
- one of the residual amount members (40, 2) surrounds the other;
- the first residual amount member (40; 44) has the dose scale, for example at an outer peripheral surface; and
- the housing (1, 2) forms the second residual amount member.

7. An administration device according to one of the preceding claims and further including a dose display (6, 25) which always displays the set product dose and for example has a further dose scale.

8. An administration device according to the preceding claim and at least one of the following features:
- the further dose scale can be read off through a sight window (7), for example a sight window (6) of the housing (1, 2);
- one of the dosing member (25) and the housing (1, 2) has the further dose scale, for example the dosing member (25), and the other a marker which for example a sight window (6) forms, wherein the set product dose can be read off at the position that the marker and the dose scale assume relative to each other; and
- the set dose and the residual amount that can still be expelled are simultaneously visible along an axis parallel to the advance direction (V) at an axial spacing from each other at positions that are stationary relative to the housing (1, 2) on the same side of the housing (1, 2) and thus without rotation of the housing (1, 2).

9. An administration device according to one of the two preceding claims **characterised in that** the set dose can be read off through a first sight window (6) and the residual amount that can still be expelled can be read off through a second sight window (7; 43) which is spaced from the first sight window (6), for example axially spaced.

10. An administration device according to one of the preceding claims **characterised in that**
(i) the housing (1, 2) is transparent in the region of the receiving means, for example it has a sight window (4),
(ii) a further dose scale (5) extends in the region of the receiving means in the advance direction (V),
(iii) and in the transparent region it is possible to see the position of a piston (10) delivering the product relative to the further dose scale (5),
(iv) so that the piston (10) forms for the further dose scale (5) a marker which against the further dose scale (5) displays the residual amount that can still be expelled.

11. An administration device according to one of the preceding claims **characterised in that** the piston rod (11) has a residual amount abutment (13), against which a residual amount counterpart abutment (14a) of the driven member (14) comes into abutment contact which limits the dosing stroke of the driven member (14) if the residual amount that can still be expelled is less than the set product dose.

12. An administration device according to one of claims 1 and 6 to 11 and further at least one of the following features:
- the first residual amount member (44) is axially immovably coupled to the driven member (14);
- the first residual amount member (44) is formed in one piece with the driven member (14) or for example is fixed to the driven member (14);
- the first residual amount member (44) projects from the driven member (14) in the advance direction (V);
- the first residual amount member (44) extends in the advance direction (V) into the receiving means of the housing (1, 2) or into axially overlapping relationship with the receiving means; and
- the first residual amount member (44) is in the form of a bar.

13. An administration device according to one of the preceding claims and at least one of the following features:
- the driven member (14) is coupled in entrainment engagement with the piston rod (11) in the advance direction (V) and entrains the piston rod (11) in the expulsion stroke;
- the piston rod (11) has an axially extended row of teeth (12) with saw teeth and the driven member (14) is coupled to the piston rod (11) by a tooth engagement involving an entrainment member (15) and the row of teeth (12) to move the piston rod (11) in the advance direction (V) in the expulsion stroke, wherein the entrainment member (15) is axially immovably coupled to the driven member (14), for example being formed on the driven member (14);
- the piston rod (11) is prevented from a movement in opposite relationship to the advance direction (V); and
- the driven member (14) is movable only in and in opposite relationship to the advance direction (V).

14. An administration device according to one of the preceding claims and at least one of the following features:
- in the selection positions the dosing member (25) determines the dosing stroke by abutment contact, wherein the driven member (14) for example comes directly into abutment contact;
- the dosing member (25) forms for each of the selection positions a dosing abutment (26) and determines the dosing stroke by abutment contact;
- the dosing member (25) is rotatable relative to the housing (1, 2) about an axially extended axis of rotation (L) and has distributed around the axis of rotation (L) a plurality of dosing abutments (26) at axially different heights or a dosing abutment which extends continuously in a spiral form around the axis of rotation (L), the abutment or abutments predetermining the dosing stroke by abutment contact in accordance with the selection position of the dosing member (25);
- the dosing member (25) is axially immobile relative to the housing (1, 2); and
- the dosing member (25) is a sleeve body which surrounds the driven member (14) or a drive member (20) for the driven member (14).

15. An administration device according to one of the preceding claims and further including:
(i) a drive member (20) which is movable by means of an axial stroke from an expulsion position into a triggering position and from same back into the expulsion position,
(ii) wherein the drive member (20) in at least one of the selection positions of the dosing member (25) performs a longer stroke than the driven member (14), for example being movable axially independently of the driven member (24), and
(iii) a coupling (18, 22; 31) which so couples the drive member (20) to the driven member (14) that also for the at least one of the selection positions the stroke of the drive member (20) into the triggering position causes the dosing stroke and the stroke of the drive member (20) into the expulsion position causes the expulsion stroke of the driven member (14).

## Revendications

1. Dispositif d'administration comportant une indication de quantité résiduelle, le dispositif comprenant :
a) un boîtier (1, 2) présentant un logement pour un produit à administrer,
b) une tige de piston (11) qui est mobile pour distribuer une dose de produit dans une direction d'avancée (V),
c) un élément doseur (25) qui peut être décalé pour l'ajustement de la dose de produit, en diverses positions choisies par rapport au boîtier (1, 2),
d) un élément d'entraînement de sortie (14) qui peut exécuter contre la direction d'avancée (V) une course de dosage jusqu'à une position de dosage préétablie au moyen de l'élément de dosage (25) et qui peut exécuter à partir de la position de dosage, une course de distribution dans la direction d'avancée (V),
e) l'élément d'entraînement de sortie (14) étant couplé à la tige de piston (11) de telle sorte que la course de distribution entraîne le mouvement de la tige de piston (11) dans la direction d'avancée (V),
f) un premier élément de quantité résiduelle (40 ; 44) qui est couplé à l'élément d'entraînement de sortie (14) de telle sorte que la course de dosage entraîne un mouvement du premier élément de quantité résiduelle (40 ; 44) dans une première direction et la course de distribution entraîne un mouvement dans la direction opposée,
g) et un deuxième élément de quantité résiduelle (2 ; 1) par rapport auquel le premier élément de quantité résiduelle (40 ; 44) exécute les mouvements,
h) l'un (40 ; 44) des éléments de quantité résiduelle présentant une graduation de dosage (42 ; 45), et à partir de la position qui reçoit l'autre (2 ; 1) élément de quantité résiduelle par rapport à la graduation de dosage (42 ; 45), la quantité résiduelle encore distribuable du produit étant lisible,
**caractérisé en ce que**
i) le dispositif d'administration comprend en outre une commande à came (14, 17, 40, 41) couplant le premier élément de quantité résiduelle (40) et l'élément d'entraînement de sortie (14), laquelle commande à came présente un élément de guidage comportant une came de guidage (41) et un membre d'engagement avec un élément d'engagement (17) se trouvant dans un engagement de guidage (17, 40) avec la came de guidage (41), la commande à came (14, 17, 40, 41) convertissant la course de dosage et la course de distribution de l'élément d'entraînement de sortie (14) en des mouvements du premier élément de quantité résiduelle (40), par exemple cet engagement de guidage (17, 41) convertissant déjà uniquement les courses en des mouvements du premier élément de quantité résiduelle (40).

2. Dispositif d'administration selon la revendication précédente, dans lequel la commande (14, 17, 40, 41) transforme la course de dosage et la course de distribution de l'élément d'entraînement de sortie (14) en fonction de la longueur.

3. Dispositif d'administration selon la revendication précédente et au moins l'une des caractéristiques suivantes :
- l'élément de guidage et l'élément d'engagement forment dans l'engagement de guidage (17, 41) une articulation à vis qui présente, par rapport à son axe de rotation (L), un pas de vis qui est tel qu'une force axiale agissant dans l'engagement de guidage (17, 41) entraîne une rotation relative entre l'élément de guidage et l'élément d'engagement ;
- le premier élément de quantité résiduelle (40) s'étend autour de l'élément d'entraînement de sortie (14) et est dans une zone périphérique interne tournée vers l'élément d'entraînement de sortie (14) dans l'engagement de guidage (17, 41), par exemple avec l'élément d'entraînement de sortie (14) ;
- le premier élément de quantité résiduelle (40) s'étend autour de l'élément d'entraînement de sortie (14) et l'élément d'entraînement de sortie (14) est dans une zone périphérique extérieure tournée vers le premier élément de quantité résiduelle (40) dans l'engagement de guidage (17, 41), par exemple avec le premier élément de quantité résiduelle (40) ;
- le premier élément de quantité résiduelle (40) peut tourner autour d'un axe de rotation (L) et forme dans une zone périphérique interne s'étendant autour de l'axe de rotation (L) soit l'élément d'engagement soit l'élément de guidage de la commande à came (17, 41).

4. Dispositif d'administration selon l'une quelconque des trois revendications précédentes et au moins l'une des caractéristiques suivantes :
- l'élément d'entraînement de sortie (14) et le premier élément de quantité résiduelle (40) forment la commande (14, 17, 40, 41), de préférence la commande à came (14, 17, 40, 41) selon l'une quelconque des deux revendications précédentes ;
- le premier élément de quantité résiduelle (40) peut tourner autour de l'axe de rotation (L) ;
- le premier élément de quantité résiduelle (40) est en forme de gaine.

5. Dispositif d'administration selon l'une quelconque des revendications 1 à 3, dans lequel le premier élément de quantité résiduelle (40) peut tourner autour d'un axe de rotation (L) et n'est pas mobile axialement relativement au boîtier (1, 2).

6. Dispositif d'administration selon l'une quelconque des revendications précédentes et au moins l'une des caractéristiques suivantes ;
- l'autre élément de quantité résiduelle (2 ; 1) présente une fenêtre de vue (7 ; 43) par laquelle la graduation de dosage est lisible ;
- l'un des éléments de quantité résiduelle (40, 2) entoure l'autre ;
- le premier élément de quantité résiduel (40 ; 44) présente la graduation de dosage, par exemple sur une surface du pourtour extérieur ;
- le boîtier (1, 2) forme le deuxième élément de quantité résiduel.

7. Dispositif d'administration selon l'une quelconque des revendications précédentes, comprenant en outre une indication de dose (6, 25) qui indique toujours la dose de produit ajustée et par exemple, une autre graduation de dose.

8. Dispositif d'administration selon la revendication précédente et au moins l'une des caractéristiques suivantes :
- l'autre graduation de dose est lisible par une fenêtre de vue (7) par exemple, fenêtre de vue (6) du boîtier (1, 2) ;
- l'un des éléments parmi l'élément de dosage (25) et le boîtier (1, 2) présente l'autre graduation de dosage, par exemple l'élément de dosage (25) et l'autre, un marqueur qui forme par exemple une fenêtre de vue (6), la dose de produit ajustée pouvant être lue sur la position que prennent le marqueur et la graduation de dosage l'un par rapport à l'autre ;
- la dose ajustée et la quantité résiduelle encore à distribuer sont le long d'un axe parallèle à la direction d'avancée (V) à un intervalle axial l'une de l'autre sur des positions fixes relativement au boîtier (1, 2), du même côté du boîtier (1, 2) et sont ainsi visibles en même temps sans tourner le boîtier (1, 2).

9. Dispositif d'administration selon l'une quelconque des deux revendications précédentes, **caractérisé en ce que** la dose ajustée est lisible par une première fenêtre de vue (6) et la quantité résiduelle encore à distribuer est lisible par une deuxième fenêtre de vue (7 ; 43) à distance de la première fenêtre de vue (6), par exemple à distance axiale.

10. Dispositif d'administration selon l'une quelconque des revendications précédentes, **caractérisé en ce que**
(i) le boîtier (1, 2) est transparent dans la zone de logement , et présente par exemple une fenêtre de vue (4),
(ii) dans la zone du logement dans la direction d'avancée (V) s'étend une autre graduation (5),
(iii) et dans la zone transparente, la position d'un piston (10) acheminant le produit par rapport à l'autre graduation de dosage (5) est reconnaissable,
(iv) de sorte que le piston (10) forme pour l'autre graduation de dosage (5) un marqueur qui indique sur l'autre graduation de dosage (5) la quantité résiduelle encore à distribuer.

11. Dispositif d'administration selon l'une quelconque des revendications précédentes, **caractérisées en ce que** la tige de piston (11) présente une butée de quantité résiduelle (13) contre laquelle parvient une contre-butée de quantité résiduelle (14a) de l'élément d'entraînement de sortie (14) dans un contact de butée qui limite la course de dosage de l'élément d'entraînement de sortie (14) lorsque la quantité résiduelle encore à distribuer est inférieure à la dose de produit ajustée.

12. Dispositif d'administration selon l'une quelconque des revendications 1 et 6 à 11 et en outre au moins l'une des caractéristiques suivantes :
- le premier élément de quantité résiduelle (44) est couplé axialement à l'élément d'entraînement de sortie (14) de façon non mobile ;
- le premier élément de quantité résiduelle (44) est formé d'un seul tenant avec l'élément d'entraînement de sortie (14) ou par exemple est fixé à l'élément d'entraînement de sortie (14) ;
- le premier élément de quantité résiduelle (44) dépasse de l'élément d'entraînement de sortie (14) dans la direction d'avancée (V) ;
- le premier élément de quantité résiduelle (44) s'étend dans la direction d'avancée (V) dans le logement du boîtier (1, 2) ou dans un chevauchement axial avec le logement ;
- le premier élément de quantité résiduelle (44) est en forme de tige.

13. Dispositif de distribution selon l'une quelconque des revendications précédentes et au moins l'une des caractéristiques suivantes :
- l'élément d'entraînement de sortie (14) est couplé à la tige de piston (11) dans la direction d'avancée (V) dans un engagement d'entraînement et emporte la tige de piston (11) lors de la course de distribution ;
- la tige de piston (11) présente une série de dents (12) s'étendant axialement avec des dents de scie, et l'élément d'entraînement de sortie (14) est couplé à la tige de piston (11) par un engagement de dents dans lequel se trouvent un entraîneur (15) et la série de dents (12) pour déplacer la tige de piston (11) lors de la course de distribution dans la direction d'avancée (V),
l'entraîneur (15) n'étant pas lié de façon mobile axialement à l'élément d'entraînement de sortie (14), par exemple étant formé sur l'élément d'entraînement de sortie (14) ;
- la tige de piston (11) est empêchée d'exécuter un mouvement contre la direction d'avancée (V) ;
- élément d'entraînement de sortie (14) n'est mobile que dans la direction d'avancée (V) et à l'encontre de celle-ci.

14. Dispositif d'administration selon l'une quelconque des revendications précédentes et au moins l'une des caractéristiques suivantes :
- l'élément de dosage (25) détermine, dans les positions choisies, la course de dosage par contact avec la butée, l'élément d'entraînement de sortie (14) parvenant par exemple directement en contact avec la butée ;
- l'élément de dosage (25) forme pour chacune des positions choisies, une butée de dosage (26) et détermine la course de dosage par contact avec la butée ;
- l'élément de dosage (25) peut tourner relativement au boîtier (1, 2) autour d'un axe de rotation (L) s'étendant axialement et présente autour de l'axe de rotation (L), plusieurs butées de dosage (26) réparties sur des hauteurs axiales différentes ou une butée de dosage en spirale s'étendant en continu autour de l'axe de rotation (L) qui détermine(nt) la course de dosage par contact avec la butée de façon correspondant à la position choisie de l'élément de dosage (25) ;
- l'élément de dosage (25) n'est pas mobile axialement relativement au boîtier (1, 2) ;
- l'élément de dosage (25) est un corps de gaine qui entoure l'élément d'entraînement de sortie (14) ou un élément d'entraînement d'amenée (20) pour l'élément d'entraînement de sortie (14).

15. Dispositif d'administration selon l'une quelconque des revendications précédentes, comprenant en outre
(i) un élément d'entraînement d'amenée (20) qui est mobile au moyen d'une course axiale d'une position de distribution à une position de déclenchement et à partir de celle-ci, peut revenir en position de distribution,
(ii) l'élément d'entraînement d'amenée (20) exécutant dans au moins l'une des positions choisies de l'élément de dosage (25) une course plus longue que celle de l'élément d'entraînement de sortie (14), par exemple étant mobile axialement, indépendamment de l'élément d'entraînement de sortie (14),
(iii) et un couplage (18, 22 ; 31) qui couple l'élément d'entraînement d'amenée (20) avec l'élément d'entraînement de sortie (14) de telle sorte qu'également pour au moins l'une des positions choisies, la course de l'élément d'entraînement d'amenée (20) entraîne, dans la position de déclenchement, la course de dosage et la course de l'élément d'entraînement d'amenée (20) et, dans la position de distribution, la course de distribution de l'élément d'entraînement de sortie (14).
